(19) Europäisches Patentamt
European Patent Office
Office européen des brevets

(11) EP 3 116 973 B1

(12) **EUROPÄISCHE PATENTSCHRIFT**

(45) Veröffentlichungstag und Bekanntmachung des Hinweises auf die Patenterteilung:
**19.06.2019 Patentblatt 2019/25**

(21) Anmeldenummer: **15707275.2**

(22) Anmeldetag: **19.02.2015**

(51) Int Cl.:
*C09K 11/06* (2006.01) *C07D 209/82* (2006.01)
*H01J 9/22* (2006.01) *H01L 51/00* (2006.01)
*C07D 413/14* (2006.01) *C07D 221/20* (2006.01)
*C07D 403/04* (2006.01) *C07D 403/10* (2006.01)
*C07D 401/14* (2006.01) *C07D 413/10* (2006.01)
*C07D 251/24* (2006.01) *C09D 5/22* (2006.01)
*C09D 5/24* (2006.01) *C09D 5/25* (2006.01)
*C09K 11/02* (2006.01) *H01L 51/50* (2006.01)
*C07D 403/14* (2006.01) *H01L 51/56* (2006.01)

(86) Internationale Anmeldenummer:
**PCT/EP2015/000377**

(87) Internationale Veröffentlichungsnummer:
**WO 2015/135625 (17.09.2015 Gazette 2015/37)**

(54) **FORMULIERUNGEN LUMINESZIERENDER VERBINDUNGEN**

FORMULATIONS OF LUMINESCENT COMPOUNDS

FORMULATIONS DE COMPOSÉS LUMINESCENTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.03.2014 EP 14000920**

(43) Veröffentlichungstag der Anmeldung:
**18.01.2017 Patentblatt 2017/03**

(73) Patentinhaber: **Merck Patent GmbH**
**64293 Darmstadt (DE)**

(72) Erfinder:
- **PARHAM, Amir Hossain**
  **60486 Frankfurt am Main (DE)**
- **STOESSEL, Philipp**
  **60486 Frankfurt am Main (DE)**
- **PFLUMM, Christof**
  **64291 Darmstadt (DE)**
- **JATSCH, Anja**
  **60389 Frankfurt am Main (DE)**
- **KAISER, Joachim**
  **64289 Darmstadt (DE)**
- **HAYER, Anna**
  **64293 Darmstadt (DE)**

(56) Entgegenhaltungen:
**WO-A1-2013/081088 WO-A1-2013/154064**

- **HIROKI UOYAMA ET AL: "Highly efficient organic light-emitting diodes from delayed fluorescence", NATURE, Bd. 492, Nr. 7428, 12. Dezember 2012 (2012-12-12), Seiten 234-238, XP055048388, ISSN: 0028-0836, DOI: 10.1038/nature11687**
- **JAN ZIEGLER1 ET AL: "Silica-Coated InP/ZnS Nanocrystals as Converter Material in White LEDs", ADVANCED MATERIALS, WILEY - V C H VERLAG GMBH & CO. KGAA, DE, Bd. 20, Nr. 21, 3. November 2008 (2008-11-03), Seiten 4068-4073, XP002637237, ISSN: 0935-9648, DOI: 10.1002/ADMA.200800724 [gefunden am 2008-10-13]**
- **RYOICHI ISHIMATSU ET AL: "Solvent Effect on Thermally Activated Delayed Fluorescence by 1,2,3,5-Tetrakis(carbazol-9-yl)-4,6-dicyan obenzene", THE JOURNAL OF PHYSICAL CHEMISTRY A, Bd. 117, Nr. 27, 12. Juni 2013 (2013-06-12), Seiten 5607-5612, XP055122928, ISSN: 1089-5639, DOI: 10.1021/jp404120s**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 116 973 B1

## Beschreibung

Die vorliegende Erfindung betrifft Formulierungen enthaltend lumineszierende Verbindungen mit einem geringen Singulett-Triplett Abstand, sowie deren Verwendung zur Herstellung organischer elektronischer Vorrichtungen.

Der Aufbau einer speziellen organischen elektronischen Vorrichtung, in denen organische Halbleiter als funktionelle Materialien eingesetzt werden, ist beispielsweise in US 4539507, US 5151629, EP 0676461 und WO 98/27136 offenbart. Es handelt sich dabei um organische lichtemittierende Dioden (OLED), eine spezielle Vorrichtung aus der Gruppe der organischen Elektrolumineszenzvorrichtungen. Als emittierende Materialien werden hierbei insbesondere auch metallorganische Iridium- und Platinkomplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich.

Trotz der guten Ergebnisse, die mit metallorganischen Iridum- und Platinkomplexen erzielt werden, weisen diese jedoch auch eine Reihe von Nachteilen auf: So handelt es sich bei Iridium und Platin um seltene und teure Metalle. Es wäre daher zur Ressourcenschonung wünschenswert, die Verwendung dieser seltenen Metalle vermeiden zu können. Weiterhin weisen derartige Metallkomplexe teilweise eine geringere thermische Stabilität auf, als rein organische Verbindungen, so dass auch aus diesem Grund die Verwendung rein organischer Verbindungen vorteilhaft wäre, sofern diese zu vergleichbar guten Effizienzen führen. Weiterhin sind blau, insbesondere tiefblau phosphoreszierende Iridium- bzw. Platinemitter mit hoher Effizienz und Lebensdauer technisch nur schwer zu verwirklichen, so dass es auch hier Verbesserungsbedarf gibt. Weiterhin gibt es insbesondere bei der Lebensdauer phosphoreszierender OLEDs, welche Ir- oder Pt-Emitter enthalten, Verbesserungsbedarf, wenn die OLED bei höherer Temperatur betrieben wird, wie dies für einige Anwendungen erforderlich ist. Weiterhin werden viele der bekannten organischen Halbleitermaterialien zur Herstellung elektronischer Vorrichtungen mittels Sublimation im Vakuum aufgedampft, was teilweise sehr aufwendig und kostenintensiv ist. Zudem können nicht alle Materialien aufgedampft werden, so dass bestimme organische Materialien trotz guter opto-elektronischer Eigenschaften aufgrund ihrer schlechten Prozessierbarkeit nicht in elektronischen Vorrichtungen verwendet werden können. Insbesondere für die Massenproduktion elektronischer Erzeugnisse ist eine kostengünstige Prozessierbarkeit organischer Materialien von sehr großem kommerziellen Interesse. Wünschenswert sind daher hoch effiziente organische Moleküle, die aus Lösung prozessiert werden können.

Eine Alternative zur Entwicklung phosphoreszierender Metallkomplexe ist die Verwendung von Emittern, die thermisch aktivierte verzögerte Fluoreszenz (thermally activated delayed fluorescence, TADF) zeigen (z. B. H. Uoyama et al., Nature 2012, Vol. 492, 234). Hierbei handelt es sich um organische Materialien, bei denen der energetische Abstand zwischen dem niedrigsten Triplettzustand $T_1$ und dem ersten angeregten Singulettzustand $S_1$ so klein ist, dass dieser Energieabstand kleiner oder im Bereich der thermischen Energie liegt. Aus quantenstatistischen Gründen entstehen bei elektronischer Anregung in der OLED die angeregten Zustände zu 75% im Triplettzustand und zu 25% im Singulettzustand. Da rein organische Moleküle üblicherweise nicht aus dem Triplettzustand emittieren, können 75% der angeregten Zustände nicht für die Emission genutzt werden, wodurch prinzipiell nur 25% der Anregungsenergie in Licht umgewandelt werden können. Ist jedoch der energetische Abstand zwischen dem niedrigsten Triplettzustand und dem niedrigsten angeregten Singulettzustand nicht oder nicht wesentlich größer als die thermische Energie, die durch $k_BT$ beschrieben wird, wobei $k_B$ für die Boltzmannkonstante und T für die Temperatur steht, so ist aus dem Triplettzustand durch thermische Anregung der erste angeregte Singulettzustand des Moleküls zugänglich und kann thermisch besetzt werden. Da dieser Singulettzustand ein emissiver Zustand ist, aus dem Fluoreszenz möglich ist, kann dieser Zustand zur Erzeugung von Licht verwendet werden. Somit ist prinzipiell die Umwandlung von bis zu 100 % der elektrischen Energie in Licht möglich bei der Verwendung rein organischer Materialien als Emitter. So wird im Stand der Technik eine externe Quanteneffizienz von mehr als 19% beschrieben, was in derselben Größenordnung wie für phosphoreszierende OLEDs liegt. Somit ist es mit derartigen rein organischen Materialien möglich, sehr gute Effizienzen zu erreichen und gleichzeitig die Verwendung seltener Metalle wie Iridium oder Platin zu vermeiden. Weiterhin ist es mit solchen Materialien auch möglich, hocheffiziente blau emittierende OLEDs zu erzielen.

Die im Stand der Technik beschriebenen Verbindungen, die TADF zeigen, werden durchweg mittels Sublimation im Vakuum aufgedampft, um elektronische Vorrichtungen enthaltend diese Verbindungen herzustellen.

H.Uoyama et al, Nature 2012, 234-238 offenbart eine TADF-Verbindung, die in einem Lösungsmittel gelöst wird, um Absorptions- und Photolumineszenzspektrum aufzunehmen. Allerdings offenbart D1 keine Formulierung enthaltend eine TADF-Verbindung, um eine OLED aus Lösung zu prozessieren.

J.Ziegler et al, Adv.Mater.2008, 44068-4073 offenbart Formulierungen enthaltend Quantum Dots.

Generell gibt es bei den Verbindungen aus dem Stand der Technik noch erheblichen Verbesserungsbedarf, insbesondere im Hinblick auf Prozessierbarkeit. Insbesondere die Ausfallrate bei der Herstellung von organischen elektronischen Vorrichtungen, wie den OLEDs, ist nach wie vor zu groß, was die Wirtschaftlichkeit der Herstellprozesse negativ beeinträchtigt.

Die der vorliegenden Erfindung zugrunde liegende technische Aufgabe besteht somit in der Bereitstellung stofflicher Zusammensetzungen, die eine kostengünstige Herstellung organischer elektronischer Vorrichtungen, insbesondere von OLEDs, erlauben, wobei die Vorrichtungen sehr gute Leistungsdaten aufweisen und die Ausfallraten bei der Herstellung der Vorrichtungen verbessert werden.

Überraschend wurde gefunden, dass die technische Aufgabe mittels der unten näher beschriebenen Formulierungen gelöst wird. Anhand dieser Formulierungen können Organische elektronische Vorrichtungen, insbesondere auch OLEDs, hergestellt werden, die aus Lösung prozessiert werden. Die mittels der erfindungsgemäßen Formulierungen hergestellten Vorrichtungen weisen sehr gute Leistungsdaten auf und zeigen eine deutlich verringerte Ausfallrate während des Herstellprozesses. Die erfindungsgemäßen Formulierungen beseitigen daher die im Stand der Technik bekannten Nachteile. Die elektronischen Vorrichtungen können leicht aus Lösung prozessiert werden. Die Formulierungen eignen somit für eine kostengünstige und zuverlässige Massenproduktion elektronischer Vorrichtungen.

Gegenstand der vorliegenden Erfindung ist daher eine Formulierung enthaltend wenigstens eine Art organischer lumineszierender Verbindung (TADF-Verbindung) sowie wenigstens ein organisches Lösungsmittel, dadurch gekennzeichnet, dass die TADF-Verbindung einen Abstand zwischen dem niedrigsten Triplettzustand $T_1$ und dem ersten angeregten Singulettzustand $S_1$ von kleiner oder gleich 0.15 eV aufweist, und wobei die Formulierung wenigstens eine weitere Komponente enthält, die aus den organischen Halbleitermaterialien, anorganischen Halbleitermaterialien oder aus Quantenpunkten (Quantum Dots) oder Quantenstäbchen (Quantum Rods) ausgewählt wird;
und wobei die Konzentration der TADF-Verbindung in der Formulierung bezogen auf die Gesamtformulierung im Bereich von 1 bis 20 Gew.-% liegt;
und wobei es sich bei der TADF-Verbindung um eine aromatische Verbindung handelt, die sowohl Donor- als auch Akzeptorsubstituenten aufweist, wobei die Donorsubstituenten Diaryl- oder Heteroarylaminogruppen, Carbazole, Indenocarbazole oder Indolocarbazole darstellen und die Aktzeptorgruppen Cyanogruppen, $CF_3$, Triazine, Pyrimidine, Phosphinoxide oder Ketone darstellen.

Unter Formulierung wird vorliegend eine Zusammensetzung verstanden, die neben dem wenigstens einen TADF-Molekül noch ein organisches Lösungsmittel oder organisches Lösungsmittelgemisch enthält. Das Lösungsmittel oder Lösungsmittelgemisch ist dabei bevorzugt im Überschuss vorhanden.

Unter Lösungsmittelgemisch wird vorliegend eine Mischung von wenigstens zwei unterschiedlichen Lösungsmitteln verstanden.

Weiterhin bevorzugt ist, dass die Formulierung in flüssiger Form vorliegt. Die Formulierung kann dabei eine echte Lösung, eine Emulsion, Miniemulsion, Dispersion oder Suspension darstellen, wobei ganz bevorzugt ist, wenn die Formulierung eine echte Lösung ist.

Die Formulierung wird, wie an anderer Stelle der vorliegenden Erfindung gezeigt, zur Herstellung einer Schicht organischer elektronischer Vorrichtungen, insbesondere der Emissionsschicht von OLEDs, verwendet. Während der Herstellung der Schicht wird das Lösungsmittel oder Lösungsmittelgemisch entfernt, so dass die TADF-Verbindung in der Schicht im Überschuss gegenüber dem verbleibenden Lösungsmittel oder Lösungsmittelgemisch vorliegt. Vorzugsweise findet sich das Lösungsmittel oder Lösungsmittelgemisch, wenn überhaupt, nur noch in Spuren in der Schicht der organischen elektronischen Vorrichtung. Ganz bevorzugt ist, wenn das Lösungsmittel oder Lösungsmittelgemisch vollständig entfernt wurde und daher nicht mehr in der aufgebrachten Schicht aufzufinden ist.

Die TADF-Verbindung ist eine kohlenstoffhaltige Verbindung, die keine Metalle enthält. Insbesondere ist die TADF-Verbindung eine organische Verbindung, die aus den Elementen C, H, D, B, Si, N, P, O, S, F, Cl, Br und I aufgebaut ist.

Bevorzugt weisen die TADF-Verbindung einen Abstand zwischen $S_1$ und $T_1$ von kleiner oder gleich 0.12 eV, besonders bevorzugt von kleiner oder gleich 0.10 eV, ganz besonders bevorzugt von kleiner oder gleich 0.08 eV und insbesondere bevorzugt von kleiner oder gleich 0.05 eV auf.

In einer bevorzugten Ausführungsform hat das $S_1$-Niveau der TADF-Verbindung eine höhere Energie als das $T_1$-Niveau. Der Abstand der Energienieveaus $S_1$ und $T_1$ ist dann wie folgt definiert: $\Delta E= E(S_1)-E(T_1)$, wobei $\Delta E$ kleiner oder gleich 0.15 eV ist, bevorzugt ist er kleiner oder gleich 0.12 eV, besonders bevorzugt ist er kleiner oder gleich 0.10 eV, ganz besonders bevorzugt ist er kleiner oder gleich 0.08 eV und insbesondere bevorzugt ist er kleiner oder gleich 0.05 eV.

Eine lumineszente Verbindung im Sinne der vorliegenden Erfindung ist eine Verbindung, die in der Lage ist, unter optischer Anregung in einer Umgebung, wie sie in der organischen Elektrolumineszenzvorrichtung vorliegt, bei Raumtemperatur Licht zu emittieren. Dabei weist die Verbindung bevorzugt eine Lumineszenzquanteneffizienz von mindestens 40% auf, besonders bevorzugt von mindestens 50%, ganz besonders bevorzugt von mindestens 60% und insbesondere bevorzugt von mindestens 70%. Dabei wird die Lumineszenzquanteneffizienz bestimmt in einer Schicht in Mischung mit dem Matrixmaterial, wie sie in der organischen Elektrolumineszenzvorrichtung verwendet werden soll. Wie die Bestimmung der Lumineszenzquantenausbeute im Sinne der vorliegenden Erfindung durchgeführt wird, ist im Beispielteil ausführlich allgemein beschrieben.

Weiterhin ist es bevorzugt, wenn die TADF-Verbindung eine kurze Abklingzeit aufweist. Dabei ist die Abklingzeit bevorzugt kleiner oder gleich 50 $\mu$s. Wie die Bestimmung der Abklingzeit im Sinne der vorliegenden Erfindung durchgeführt wird, ist im Beispielteil ausführlich allgemein beschrieben.

Die Energie des niedrigsten angeregten Singulettzustands ($S_1$) und des niedrigsten Triplettzustands ($T_1$) werden durch quantenchemische Rechnung bestimmt. Wie diese Bestimmung im Sinne der vorliegenden Erfindung durchgeführt wird, ist im Beispielteil ausführlich allgemein beschrieben.

Bei der TADF-Verbindung handelt es sich um eine aromatische Verbindung, die sowohl Donor- wie auch Akzeptorsubstituenten aufweist, wobei bevorzugt ist, wenn das LUMO und das HOMO der Verbindung räumlich nur schwach überlappen.

Bevorzugt weist die TADF-Verbindung eine geringe räumliche Überlappung $\Lambda$ der Molekülorbitale, die bei bestimmten elektronischen Übergängen beteiligt sind (Charge-Transfer-Zustände) auf.

Vorliegend bedeutet eine geringe Überlappung der Molekülorbitale, dass der Wert des Parameters A 0.3 oder kleiner ist, bevorzugt ist er 0.2 oder kleiner, ganz bevorzugt ist er 0.15 oder kleiner, ganz besonders bevorzugt ist er 0.1 oder kleiner und insbesondere bevorzugt ist er 0.05 oder kleiner. Wie die Bestimmung des Parameters A im Sinne der vorliegenden Erfindung durchgeführt wird, ist im Beispielteil ausführlich allgemein beschrieben.

Was unter Donor- bzw. Akzeptorsubstituenten verstanden wird, ist dem Fachmann hinlänglich bekannt. Geeignete Donorsubstituenten sind insbesondere Diaryl- bzw. -heteroarylaminogruppen sowie Carbazolgruppen bzw. Carbazolderivate, die jeweils bevorzugt über N an die aromatische Verbindung gebunden sind. Dabei können diese Gruppen auch weiter substituiert sein. Geeignete Akzeptorsubstituenten sind insbesondere Cyanogruppen, aber auch beispielsweise elektronenarme Heteroarylgruppen, die auch weiter substituiert sein können.

Unter der Akzeptorgruppe A (auch Akzeptorsubstituent genannt) wird vorliegend eine Gruppe verstanden, die eine Elektronenakzeptorgruppe darstellt. Was unter einer Akzeptorgruppe verstanden wird, ist dem Fachmann gut bekannt. Es ist bevorzugt, wenn die Akzeptorgruppe einen darstellt. Was unter einer Akzeptorgruppe verstanden wird, ist dem Fachmann gut bekannt. Es ist bevorzugt, wenn die Akzeptorgruppe einen negativen induktiven Effekt (-I) und/oder einen negativen mesomeren Effekt (-M) aufweist. Die Bestimmung der Parameter mit Hilfe der Hammett-Gleichung ist dem Fachmann auch gut bekannt Geeignete Akzeptorsubstituenten sind insbesondere Cyanogruppen, aber auch $CF_3$ und beispielsweise elektronenarme Heteroarylgruppen, die auch weiter substituiert sein können. Beispiele bevorzugter elektronenarmer Heteroarylgruppen sind ausgewählt aus der Gruppe bestehend aus Triazinen, Pyrimidinen, Phosphinoxiden und Ketonen.

Unter der Donorgruppe D (auch Donorsubstituent genannt) wird vorliegend eine Gruppe verstanden, die eine Elektronendonorgruppe darstellt. Was unter einer Donorgruppe verstanden wird, ist dem Fachmann gut bekannt. Es ist bevorzugt, wenn die Donorgruppe einen positiven induktiven Effekt (+I) und/oder einen positiven mesomeren Effekt (+M) aufweist. Die Bestimmung der Parameter mit Hilfe der Hammett-Gleichung ist dem Fachmann gut bekannt. Geeignete Donorsubstituenten sind insbesondere Diaryl- bzw. -heteroarylaminogruppen sowie Carbazolgruppen bzw. Carbazolderivate, wie Indenocarbazole oder Indolocarbazole, die jeweils bevorzugt über N an die Brücke V bzw. an die Gruppe A gebunden sind. Dabei können diese Gruppen auch weiter substituiert sein.

Beispiele für geeignete Moleküle, die TADF zeigen, sind die in der folgenden Übersicht aufgeführten Strukturen.

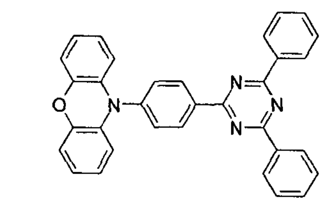

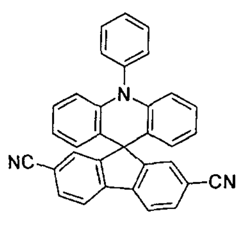

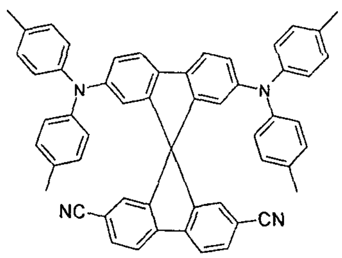

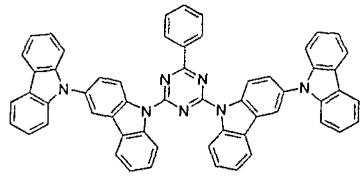

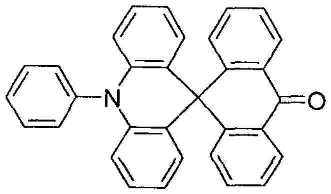

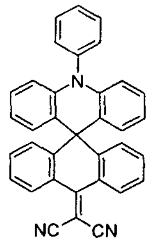

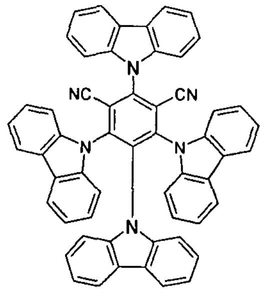

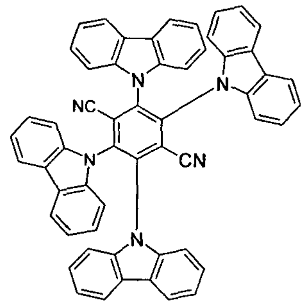

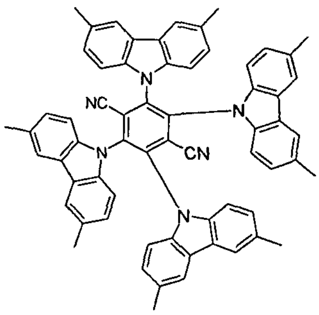

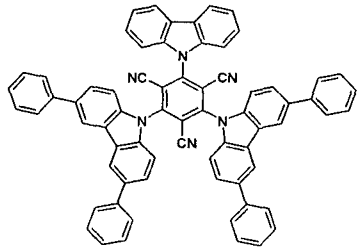

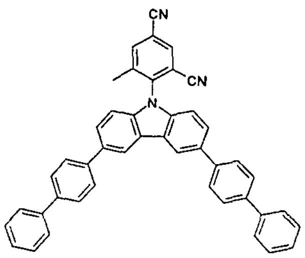

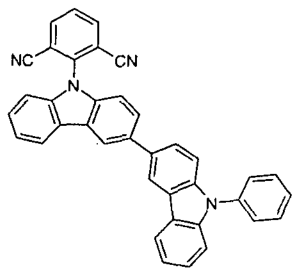

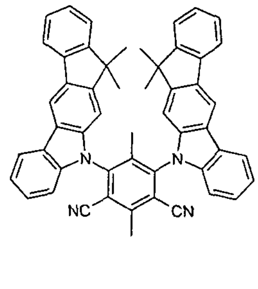

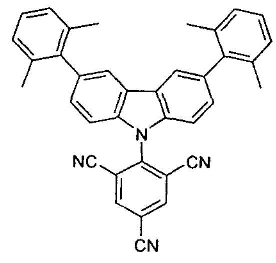

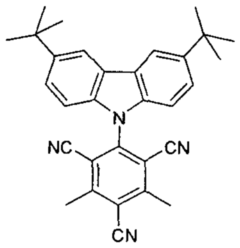

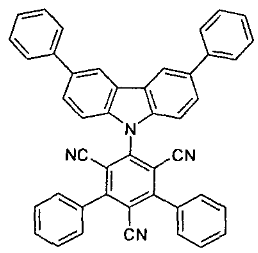

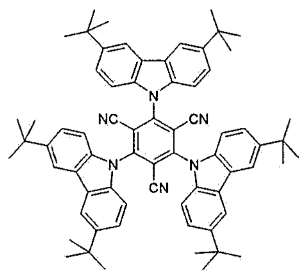

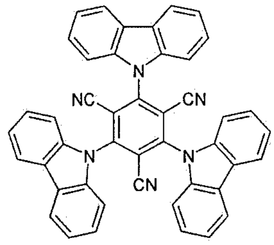

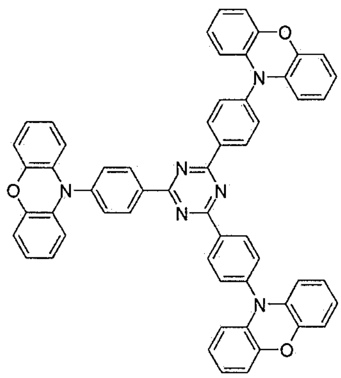

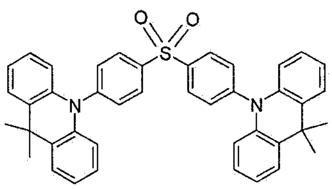

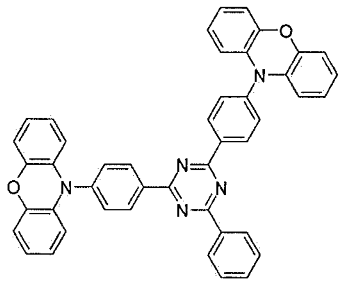

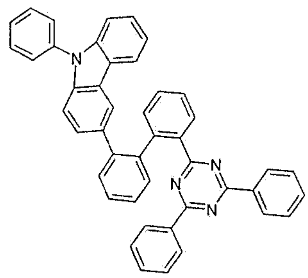

Im Stand der Technik sind eine Vielzahl von TADF-Verbindungen bekannt und es bereitet dem Fachmann keinerlei Schwierigkeiten die TADF-Verbindungen aus dem Stand der Technik auszuwählen (z.B.: Tanaka et al., Chemistry of Materials 25(18), 3766 (2013), Zhang et al., Nature Photonics advance online publication, 1 (2014), doi:10.1038/nphoton.2014.12, Serevicius et al., Physical Chemistry Chemical Physics 15(38), 15850 (2013), Youn Lee et al., Applied Physics Letters 101(9), 093306 (2012), Nasu et al., ChemComm, 49, 10385 (2013), WO 2013/154064, WO 2013/161437, WO 2013/081088, WO 2013/011954.

Bevorzugt ist, wenn das verwendetet Lösungsmittel oder die Lösungsmittel (im Fall eines Lösungsmittelgemischs) eine Oberflächenspannung von wenigstens 28 mN/m, bevorzugt wenigstens 30 mN/m, ganz bevorzugt wenigstens 32 mN/m und ganz besonders bevorzugt wenigstens 35 mN/m aufweist.

Weiterhin bevorzugt ist, wenn die Siede- oder Sublimationstemperatur des Lösungsmittels oder der Lösungsmittel kleiner als 300°C und bevorzugt weniger als 260°C beträgt.

Ganz bevorzugt ist, wenn die Viskosität des Lösungsmittel oder der verschiedenen Lösungsmittel eines Lösungsmittelgemischs größer als 3mPa*s und bevorzugt größer als 5mPa*s ist.

Weiterhin bevorzugt ist, wenn das Molekulargewicht des Lösungsmittels oder der Lösungsmittel kleiner oder gleich 1000 g/mol, bevorzugt kleiner oder gleich 700 g/mol, ganz bevorzugt kleiner oder gleich 500 g/mol und besonders

bevorzugt kleiner oder gleich 300 g/mol ist.

Wie bereits erläutert liegt das Lösungsmittel bzw. sind die Lösungsmittel eines Lösungsmittelgemischs in der Formulierung im Überschuss vor im Vergleich zu der TADF-Verbindung. Die Konzentration der TADF-Verbindung ist in der Formulierung bezogen auf die Gesamtformulierung im Bereich von 1 bis 20 Gew.-%, bevorzugt im Bereich von 3 bis 15 Gew.-% und besonders bevorzugt im Bereich von 5 bis 12 Gew.-%.

Der Fachmann kann aus einer Vielzahl von Lösungsmitteln und Lösungsmittelgemischen auswählen, um die erfindungsgemäßen Formulierungen bereitzustellen.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethyhenglycolbutylmethyl ether, Triethyleneglycolbutylmethyl ether, Diethylenglycoldibutyl ether, Triethylenglycoldimethyl ether, Diethylenglycolmonobutylether, Tripropyleneglycol dimethyl ether, Tetraethyleneglycol dimethyl ether, 2-isopropyl naphthalin, Pentylbenzol, Hexylbenzol, Heptylbenzol, octylbenzol, 1,1-Bis(3,4-Dimethylphenyl)ethan oder Mischungen diesen Lösemitteln.

Die Formulierung der vorliegenden Erfindung kann bevorzugt 0,01 bis 20 Gew.-%, ganz bevorzugt 0,05 bis 10 Gew.-%, besonders bevorzugt 0,1 bis 5 Gew.-% und insbesondere bevorzugt 0,5 bis 5 Gew.-% niedermolekulare organische Halbleitermaterialien enthalten, wobei unter niedermolekularen organischen Halbleitermaterialien (bspw. TADF-Verbindung, Matrixmaterialien, Emitter, Lochtransport- und Elektronentransportmaterialien, Loch- und Elektronenblockiermaterialien) solche verstanden werden, die ein Molekulargewicht von kleiner oder gleich 2000 g/mol, bevorzugt kleiner oder gleich 1500 g/mol, ganz bevorzugt kleiner oder gleich 1000 g/mol, ganz bevorzugt kleiner oder gleich 700 und insbesondere bevorzugt kleiner oder gleich 600 g/mol haben.

Die Formulierung der vorliegenden Erfindung enthält ein oder mehrere organische Lösungsmittel, vorzugsweise mindestens ein aromatisches Lösungsmittel. Die Lösungsmittel sind vorzugsweise aus der Gruppe bestehend aus aromatischen Kohlenwasserstoffen, wie Toluol, o-, m- oder p-Xylol, Phenoxytoluole, Trimethylbenzole (z.B. 1,2,3-, 1,2,4- und 1,3,5-Trimethylbenzole), Tetralin, anderen Mono-, Di-, Tri- und Tetraalkylbenzolen (z.B. Diethylbenzole, Methylcumol, Tetramethylbenzole usw.), aromatischen Ethern (z.B. Anisol, Alkylanisole, z.B. 2-, 3- und 4-Isomere des Methylanisols, 2,3-, 2,4-, 2,5-, 2,6-, 3,4- und 3,5-Isomere des Dimethylanisols), Naphthalinderivaten, Alkylnaphthalinderivaten (z.B. 1- und 2- Methylnaphthalin), Di- und Tetrahydronaphthalinderivaten ausgewählt. Ebenfalls bevorzugt sind aromatische Ester (z.B. Alkylbenzoate), aromatische Ketone (z.B. Acetophenon, Propiophenon), Alkylketone (z.B. Cyclohexanon), heteroaromatische Lösungsmittel (z.B. Thiophen, Mono-, Di- und Trialkylthiophene, 2-Alkylthiazole, Benzthiazole usw., Pyridine), Halogenarylene und Anilinderivate. Diese Lösungsmittel können Halogenatome enthalten.

Besonders bevorzugt sind: 3-Fluor-trifluormethylbenzol, Trifluormethylbenzol, Dioxan, Trifluormethoxybenzol, 4-Fluor-benzoltrifluorid, 3-Fluorpyridin, Toluol, 2-Fluortoluol, 2-Fluor-benzoltrifluorid, 3-Fluortoluol, Pyridin, 4-Fluortoluol, 2,5-Difluortoluol, 1-Chlor-2,4-difluorbenzol, 2-Fluorpyridin, 3-Chlorfluorbenzol, 1-Chlor-2,5-difluorbenzol, 4-Chlorfluorbenzol, Chlorbenzol, 2-Chlorfluorbenzol, p-Xylol, m-Xylol, o-Xylol, 2,6-Lutidin, 2-Fluor-m-xylol, 3-Fluor-o-xylol, 2-Chlorbenzoltrifluorid, Dimethylformamid, 2-Chlor-6-fluortoluol, 2-Fluoranisol, Anisol, 2,3-Dimethylpyrazin, Brombenzol, 4-Fluoranisol, 3-Fluoranisol, 3-Trifluormethylanisol, 2-Methylanisol, Phenetol, Benzoldioxol, 4-Methylanisol, 3-Methylanisol, 4-Fluor-3-methylanisol, 1,2-Dichlorbenzol, 2-Fluorbenzolnitril, 4-Fluorveratrol, 2,6-Dimethylanisol, Anilin, 3-Fluorbenzolnitril, 2,5-Dimethylanisol, 3,4-Dimethylanisol, 2,4-Dimethylanisol, Benzolnitril, 3,5-Dimethylanisol, N,N-Dimethylanilin, 1-Fluor-3,5-Dimethoxybenzol, Phenylacetat, N-Methylanilin, Methylbenzoat, N-Methylpyrrolidon, Morpholin, 1,2-Dihydronaphthalin, 1,2,3,4-Tetrahydronaphthalin, o-Tolunitril, Veratrol, Ethylbenzoat, N,N-Diethylanilin, Propylbenzoat, 1-Methylnaphthalin, Butylbenzoat, 2-Methylbiphenyl, 2-Phenylpyridin oder 2,2'-Bitolyl.

Besonders bevorzugt sind aromatische Kohlenwasserstoffe, insbesondere Toluol, Phenoxytoluol, Dimethylbenzole (Xylole), Trimethylbenzole, Tetralin und Methylnaphthaline, aromatische Ether, insbesondere Anisol, und aromatische Ester, insbesondere Methylbenzoat.

Insbesondere bevorzugt sind aromatische Ether, insbesondere Anisol und Derivate davon, wie Alkylanisole, und aromatische Ester, insbesondere Methylbenzoat.

Diese Lösungsmittel können als Mischung von zwei, drei oder mehr verwendet werden.

Bevorzugte organische Lösungsmittel können Löslichkeitsparameter nach Hansen von $H_d$ im Bereich von 17,0 bis 23,2 $MPa^{0,5}$, $H_p$ im Bereich von 0,2 bis 12,5 $MPa^{0,5}$ und $H_h$ im Bereich von 0,9 bis 14,2 $MPa^{0,5}$ aufweisen. Besonders bevorzugte organische Lösungsmittel weisen Löslichkeitsparameter nach Hansen von $H_d$ im Bereich von 18,5 bis 21,0 $MPa^{0,5}$, $H_p$ im Bereich von 2,0 bis 6,0 $MPa^{0,5}$ und $H_h$ im Bereich von 2,0 bis 6,0 $MPa^{0,5}$ auf.

Tabelle 2: Löslichkeitsparameter nach Hansen von brauchbaren Lösungsmitteln

| **Lösungsmittel** | $H_d$ [MPa$^{0,5}$] | $H_h$ [MPa$^{0,5}$] | $H_p$ [MPa$^{0,5}$] |
|---|---|---|---|
| 1,2,3,4-Tetrahydro-1-naphthol | 19,6 | 9,2 | 12,8 |
| 1,2,3,4-Tetrahydronaphthalin | 19,1 | 2,3 | 4,0 |
| 1,2,3,4-Tetramethylbenzol | 18,7 | 1,8 | 1,6 |
| 1,2,3,5-Tetramethylbenzol | 18,7 | 1,8 | 1,6 |
| 1,2,3-Trimethylbenzol | 19,0 | 2,9 | 1,6 |
| 1,2,4,5-Tetramethylbenzol | 18,7 | 1,8 | 1,6 |
| 1,2,4-Trichlorbenzol | 20,5 | 6,9 | 2,7 |
| 1,2,4-Trimethylbenzol | 19,0 | 2,9 | 1,6 |
| 1,2-Dihydronaphthalin | 20,1 | 5,5 | 4,9 |
| 1,2-Dimethylnaphthalin | 21,0 | 1,7 | 5,2 |
| 1,3,3-Trimethyl-2-methylenindol | 17,9 | 1,0 | 3,0 |
| 1,3-Benzodioxol | 19,7 | 7,4 | 7,9 |
| 1,3-Diisopropylbenzol | 17,5 | 0,2 | 1,1 |
| 1,3-Dimethylnaphthalin | 21,0 | 1,7 | 5,2 |
| 1,4-Benzodioxan | 19,5 | 8,7 | 7,2 |
| 1,4-Diisopropylbenzol | 17,5 | 0,6 | 1,6 |
| 1,4-Dimethylnaphthalin | 21,0 | 1,7 | 5,2 |
| 1,5-Dimethyltetralin | 19,3 | 5,5 | 2,6 |
| 1-Benzothiophen | 19,7 | 12,3 | 6,3 |
| 1-Bromnaphthalin | 23,1 | 10,3 | 6,1 |
| 1-Chlormethylnaphthalin | 22,1 | 9,9 | 5,3 |
| 1-Ethylnaphthalin | 20,7 | 7,8 | 4,4 |
| 1-Methoxynaphthalin | 21,4 | 10,5 | 7,5 |
| 1-Methylnaphthalin | 21,7 | 8,4 | 4,5 |
| 1-Methylindan | 19,4 | 5,7 | 2,5 |
| 1-Methylindol | 19,2 | 8,1 | 10,2 |
| 2,3,3-Trimethoxyindolenin | 19,6 | 6,8 | 4,2 |
| 2,3-Benzofuran | 21,3 | 5,5 | 5,6 |
| 2,3-Dihydrobenzofuran | 19,9 | 9,5 | 6,6 |
| 2,3-Dimethylanisol | 18,9 | 4,6 | 4,5 |
| 2,4-Dimethylanisol | 18,9 | 4,6 | 4,5 |
| 2,5-Dimethylanisol | 18,9 | 4,6 | 4,5 |
| 2,6-Diisopropylnaphthalin | 18,3 | 3,5 | 2,2 |
| 2,6-Dimethylanisol | 18,9 | 4,6 | 4,5 |
| 2,6-Dimethylnaphthalin | 20,1 | 5,0 | 3,0 |
| 2-Brom-3-(brommethyl)thiophen | 19,3 | 7,3 | 6,6 |
| 2-Brommethylnaphthalin | 22,0 | 9,4 | 7,2 |

(fortgesetzt)

| **Lösungsmittel** | $H_d$ [$MPa^{0,5}$] | $H_h$ [$MPa^{0,5}$] | $H_p$ [$MPa^{0,5}$] |
|---|---|---|---|
| 2-Bromnaphthalin | 23,1 | 10,3 | 6,1 |
| 2-Ethoxynaphthalin | 20,5 | 10,0 | 7,0 |
| 2-Ethylnaphthalin | 20,7 | 7,8 | 4,4 |
| 2-Isopropylanisol | 17,7 | 4,3 | 5,4 |
| 2-Methylchinolin | 20,0 | 7,8 | 4,0 |
| 2-Methylanisol | 18,3 | 5,1 | 6,2 |
| 2-Methylindol | 17,8 | 9,7 | 4,8 |
| 2-Phenoxyethanol | 18,7 | 8,5 | 13,0 |
| 3,4-Dimethylanisol | 18,9 | 4,6 | 4,5 |
| 3,5-Dimethylanisol | 18,9 | 4,6 | 4,5 |
| 3-Bromchinolin | 21,4 | 8,7 | 5,1 |
| 3-Isopropylbiphenyl | 19,1 | 1,3 | 1,9 |
| 3-Methylanisol | 18,7 | 5,7 | 5,4 |
| 4-Benzylaceton | 18,3 | 8,8 | 5,0 |
| 4-Isopropylbiphenyl | 19,0 | 2,5 | 1,9 |
| 4-Methoxybenzylalkohol | 19,0 | 8,5 | 13,3 |
| 4-Methylanisol | 18,6 | 5,9 | 7,2 |
| 4-Phenyl-2-butanon | 18,3 | 8,8 | 5,0 |
| 5,6,7,8-Tetrahydro-1-naphthol | 19,6 | 7,2 | 10,9 |
| 5,6,7,8-Tetrahydro-2-naphthol | 19,6 | 7,2 | 10,9 |
| 5,6,7,8-Tetrahydro-2-naphthylamin | 20,1 | 7,9 | 8,6 |
| 5,6.7,8-Tetrahydro-1-naphthylamin | 20,1 | 7,9 | 8,6 |
| 5-Decanolid | 17,1 | 7,8 | 3,8 |
| 5-Methoxyindan | 19,8 | 9,8 | 4,0 |
| 5-Methoxyindol | 17,4 | 12,3 | 7,8 |
| 5-tert.-Butyl-m-xylol | 17,6 | 3,4 | 2,2 |
| 6-Methoxy-1,2,3,4-tetrahydronapthalin | 19,4 | 6,8 | 5,4 |
| 6-Methylchinolin | 21,7 | 8,4 | 4,5 |
| 8-Methylchinolin | 21,7 | 8,4 | 4,5 |
| Acetophenon | 18,8 | 10,8 | 5,5 |
| Anisol | 18,5 | 5,5 | 5,2 |
| $\alpha$-Pinen | 17,4 | 3,0 | 3,2 |
| Benzonitril | 19,2 | 11,9 | 4,7 |
| Benzothiazol | 21,3 | 5,5 | 5,6 |
| Benzylacetat | 18,2 | 7,3 | 6,4 |
| Benzylalkohol | 19,1 | 6,7 | 14,2 |
| Brombenzol | 19,8 | 7,6 | 4,3 |
| Butylbenzol | 17,6 | 2,6 | 1,7 |

(fortgesetzt)

| **Lösungsmittel** | $H_d$ [MPa$^{0,5}$] | $H_h$ [MPa$^{0,5}$] | $H_p$ [MPa$^{0,5}$] |
|---|---|---|---|
| Butylbenzoat | 17,7 | 5,9 | 5,2 |
| Cyclohexylbenzol | 18,6 | 1,0 | 1,6 |
| Decahydronaphthalin | 17,5 | 0,4 | 1,0 |
| Diphenylether | 19,9 | 2,9 | 3,3 |
| Ethylphenylketon (Propiophenon) | 18,3 | 8,9 | 5,3 |
| Ethylbenzol | 18,2 | 2,7 | 2,1 |
| Ethylbenzoat | 18,1 | 6,6 | 5,9 |
| Furfurylalkohol | 18,1 | 6,7 | 11,9 |
| gamma-Terpinen | 18,0 | 2,5 | 2,8 |
| Hexylbenzol | 17,4 | 2,9 | 1,6 |
| Indan | 19,7 | 7,3 | 5,8 |
| Inden | 20,3 | 4,4 | 5,4 |
| Isoamylbenzol | 17,1 | 3,7 | 1,8 |
| Isobutylbenzol | 17,1 | 2,9 | 1,6 |
| Isopropylbenzol (Cumol) | 17,8 | 2,0 | 1,1 |
| m-Cymol | 18,1 | 2,0 | 2,1 |
| Mesitylen | 19,0 | 2,9 | 1,6 |
| Methylbenzoat | 18,5 | 7,9 | 6,4 |
| Methylphenylacetat | 18,2 | 7,3 | 6,4 |
| m-Xylol | 18,8 | 3,1 | 2,7 |
| n-Butoxybenzol | 17,5 | 4,4 | 4,1 |
| n-Butylbenzol | 17,6 | 2,6 | 1,7 |
| n-Propylbenzoat | 17,8 | 6,6 | 6,3 |
| n-Propylbenzol | 17,8 | 3,4 | 2,8 |
| o-Dichlorbenzol | 19,5 | 8,7 | 3,3 |
| o-Diethylbenzol | 17,7 | 0,7 | 1,9 |
| o-Ethyltoluol | 18,0 | 1,9 | 2,8 |
| o-Xylol | 18,4 | 2,0 | 2,9 |
| Pentylbenzol | 17,4 | 3,0 | 1,8 |
| p-Ethyltoluol | 18,3 | 3,5 | 2,8 |
| Phenetol | 18,1 | 4,6 | 4,6 |
| Phenylacetat | 18,5 | 7,9 | 6,4 |
| p-Isopropyltoluol (p-Cymol) | 18,0 | 2,5 | 2,8 |
| Propiophenon | 18,3 | 8,9 | 5,3 |
| Propylbenzoat | 17,8 | 6,6 | 6,3 |
| p-Xylol | 18,7 | 3,3 | 3,3 |
| sec.-Butylbenzol | 17,2 | 2,2 | 1,6 |
| t-Butylbenzol | 17,2 | 1,3 | 2,9 |

(fortgesetzt)

| **Lösungsmittel** | $H_d$ [$MPa^{0,5}$] | $H_h$ [$MPa^{0,5}$] | $H_p$ [$MPa^{0,5}$] |
|---|---|---|---|
| Tetralin | 19,1 | 2,3 | 4,0 |
| Thiophen | 18,8 | 5,2 | 7,4 |
| Toluol | 18,6 | 4,0 | 2,2 |
| Veratrol | 18,2 | 6,3 | 7,5 |
| $H_d$ bezieht sich auf den Dispersionsbeitrag<br>$H_p$ bezieht sich auf den polaren Beitrag<br>$H_h$ bezieht sich auf den Wasserstoffbindungsbeitrag | | | |

Vorzugsweise besitzt das Lösungsmittel bei dem verwendeten Druck, ganz bevorzugt bei Atmosphärendruck (1013 hPa), einen Siedepunkt oder eine Sublimationstemperatur von < 300°C, besonders bevorzugt ≤ 260°C, insbesondere bevorzugt ≤ 220°C. Die Verdampfung kann auch beschleunigt werden, z.B. durch Einsatz von Wärme und/oder vermindertem Druck. Durch die Verwendung von Lösungsmitteln mit einem Siedepunkt von mindestens 100°C, vorzugsweise mindestens 130°C lassen sich unerwartete Verbesserungen erreichen.

Üblicherweise kann das organische Lösungsmittel eine Oberflächenspannung von mindestens 28 mN/m, vorzugsweise mindestens 30 mN/m, besonders bevorzugt mindestens 32 mN/m und insbesondere bevorzugt mindestens 35 mN/m aufweisen. Die Oberflächenspannung kann mit einem Kontaktwinkel-Goniometer FTA (First Ten Angstrom) 125 bei 25°C gemessen werden. Einzelheiten zum Verfahren sind bei First Ten Angstrom, wie von Roger P. Woodward, Ph.D., "Surface Tension Measurements Using the Drop Shape Method" veröffentlicht, erhältlich. Vorzugsweise kann die Pendant-Drop-Methode zur Bestimmung der Oberflächenspannung verwendet werden.

Für eine grobe Schätzung kann die Oberflächenspannung unter Verwendung der Löslichkeitsparameter nach Hansen nach der in Hansen Solubility Parameters: A User's Handbook, zweite Auflage, C. M. Hansen (2007), Taylor and Francis Group, LLC (HSPiP-Handbuch) dargelegten Formel berechnet werden.

$$\text{Oberflächenspannung} = 0{,}0146 \text{ x}(2{,}28\text{x } \delta H_d^2 + \delta H_p^2 + \delta H_h^2) \text{ x MVol}^{0,2},$$

wobei:

$H_d$ sich auf den Dispersionsbeitrag bezieht,
$H_p$ sich auf den polaren Beitrag bezieht,
$H_h$ sich auf den Wasserstoffbindungsbeitrag bezieht,
MVol sich auf das molare Volumen bezieht.

Die Löslichkeitsparameter nach Hansen können nach dem Programm Hansen Solubility Parameters in Practice (HSPiP) (2. Auflage) wie bei Hanson und Abbot et al. erhältlich bestimmt werden.

Vorzugsweise kann das Lösungsmittel eine relative Verdampfungsgeschwindigkeit (Butylacetat = 100) von mindestens 0,01, vorzugsweise von mindestens 0,1, bevorzugt von mindestens 0,5, besonders bevorzugt von mindestens 5, ganz besonders bevorzugt von mindestens 10 und insbesondere bevorzugt von mindestens 20 aufweisen. Die relative Verdampfungsgeschwindigkeit kann nach DIN 53170: 2009-08 bestimmt werden. Für eine grobe Schätzung kann die relative Verdampfungsgeschwindigkeit unter Verwendung der Löslichkeitsparameter nach Hansen mit dem HSPiP-Programm wie vor- und nachstehend genannt berechnet werden.

Die Formulierung der vorliegenden Erfindung enthält vorzugsweise mindestens 70 Gew.-%, besonders bevorzugt mindestens 80 Gew.-% und insbesondere bevorzugt mindestens 90 Gew.-% eines oder mehrerer organischer Lösungsmittel.

Bei einer weiteren bevorzugten Ausführungsform enthält die Formulierung der vorliegenden Erfindung mindestens ein Polymermaterial als inertes Bindemittel. Das bedeutet, dass das Polymer keine Halbleitereigenschaften besitzt oder mit einer der Halbleiterverbindungen in der Zusammensetzung chemisch reagiert. Die geringen Leitungseigenschaften des inerten polymeren Bindemittels können als niedrige Dielektrizitätskonstante bestimmt werden. Bevorzugte Bindemittel gemäß der vorliegenden Erfindung sind Materialien mit niedriger Dielektrizitätskonstante, also solche, die bei 1.000 Hz eine Dielektrizitätskonstante (ε) von 3,3 oder weniger besitzen. Das organische Bindemittel besitzt bei 1.000 Hz vorzugsweise eine Dielektrizitätskonstante von weniger als 3,0, besonders bevorzugt 2,9 oder weniger. Vorzugsweise besitzt das organische Bindemittel bei 1.000 Hz eine Dielektrizitätskonstante von größer 1,7.

Mit besonderem Vorzug liegt die Dielektrizitätskonstante des Bindemittels im Bereich von 2,0 bis 2,9. Der Ausdruck "chemisch reagieren" wie vor- und nachstehend verwendet bezeichnet eine mögliche Oxidation oder andere chemische Reaktion des nicht leitenden Zusatzes mit den organischen lichtemittierenden Materialien und/oder Ladungstransportmaterialien unter den für die Herstellung, die Lagerung, den Transport und/oder die Verwendung der Formulierung und der OLED-Vorrichtung verwendeten Bedingungen.

Vorzugsweise weist das polymere Bindemittel ein gewichtsmittleres Molekulargewicht im Bereich von 1.000 bis 50.000.000 g/mol, besonders bevorzugt 1.500 bis 10.000.000 g/mol und insbesondere bevorzugt 2.000 bis 5.000.000 g/mol auf. Mit Polymeren mit einem gewichtsmittleren Molekulargewicht von vorzugsweise $\geq$ 10.000 g/mol, besonders bevorzugt $\geq$ 100.000 g/mol lassen sich überraschende Effekte erzielen.

Das Polymer kann insbesondere einen Polydispersitätsindex $M_w/M_n$ im Bereich von 1,0 bis 10,0, besonders bevorzugt im Bereich von 1,1 bis 5,0 und insbesondere bevorzugt im Bereich von 1,2 bis 3 besitzen.

Üblicherweise ist das polymere Bindemittel im Lösungsmittel der vorliegenden Zusammensetzung wie vor- und nachstehend beschrieben dispergierbar oder löslich. Vorzugsweise ist das polymere Bindemittel in dem organischen Lösungsmittel löslich und beträgt die Löslichkeit des polymeren Bindemittels in dem Lösungsmittel mindestens 1 g/l, besonders bevorzugt mindestens 5 g/l und insbesondere bevorzugt mindestens 10 g/l.

Gemäß einer besonderen Ausführungsform der vorliegenden Erfindung kann die Zusammensetzung vorzugsweise 0,1 bis 10 Gew.-%, besonders bevorzugt 0,25 bis 5 Gew.-% und insbesondere bevorzugt 0,5 bis 4 Gew.-% an polymerem Bindemittel enthalten.

Gemäß einer besonderen Ausführungsform enthalten die polymeren Bindemittel vorzugsweise von Styrol und/oder Olefinen abgeleitete Wiederholungseinheiten. Bevorzugte polymere Bindemittel können mindestens 80 Gew.-%, vorzugsweise 90 Gew.-% und besonders bevorzugt 99 Gew.-% von Styrolmonomeren und/oder Olefinen abgeleitete Wiederholungseinheiten enthalten.

Styrolmonomere sind in der Technik gut bekannt. Diese Monomere umfassen Styrol, substituierte Styrole mit einem Alkylsubstituenten in der Seitenkette, wie $\alpha$-Methylstyrol und $\alpha$-Ethylstyrol, substituierte Styrole mit einem Alkylsubstituenten am Ring, wie Vinyltoluol und p-Methylstyrol, halogenierte Styrole wie Monochlorstyrole, Dichlorstyrole, Tribromstyrole und Tetrabromstyrole.

Olefine sind Monomere, die aus Wasserstoff- und Kohlenstoffatomen bestehen. Diese Monomere umfassen Ethylen, Propylen, Butylen, Isopren und 1,3-Butadien.

Gemäß einem besonderen Aspekt der vorliegenden Erfindung handelt es sich bei dem polymeren Bindemittel um Polystyrol mit einem gewichtsmittleren Molekulargewicht im Bereich von 50.000 bis 2.000.000 g/mol, vorzugsweise 100.000 bis 750.000 g/mol, besonders bevorzugt im Bereich von 150.000 bis 600.000 g/mol und insbesondere bevorzugt im Bereich von 200.000 bis 500.000 g/mol.

Gemäß einer weiteren Ausführungsform der vorliegenden Erfindung handelt es sich bei dem polymeren Bindemittel um Poly-4-methylstyrol mit einem gewichtsmittleren Molekulargewicht im Bereich von 40.000 bis 120.000 g/mol, besonders bevorzugt im Bereich von 60.000 bis 100.000 g/mol.

Insbesondere kann es sich bei dem Bindemittel um Poly-$\alpha$-methylstyrol mit einem gewichtsmittleren Molekulargewicht im Bereich von 1.000 bis 20.000 g/mol, besonders bevorzugt im Bereich von 1.500 bis 6.000 g/mol handeln.

Brauchbare und bevorzugte polymere Bindemittel weisen Löslichkeitsparameter nach Hansen von $H_d$ im Bereich von 15,7 bis 23,0 $MPa^{0,5}$, $H_p$ im Bereich von 0,0 bis 20,0 $MPa^{0,5}$ und $H_h$ im Bereich von 0,0 bis 12,5 $MPa^{0,5}$ auf. Besonders bevorzugte polymere Bindemittel weisen Löslichkeitsparameter nach Hansen von $H_d$ im Bereich von 17,0 bis 21,0 $MPa^{0,5}$, $H_p$ im Bereich von 1,0 bis 5,0 $MPa^{0,5}$ und $H_h$ im Bereich von 2,0 bis 10,0 $MPa^{0,5}$ auf. Insbesondere bevorzugte polymere Bindemittel weisen Löslichkeitsparameter nach Hansen von $H_d$ im Bereich von 19,0 bis 21,0 $MPa^{0,5}$, $H_p$ im Bereich von 1,0 bis 3,0 $MPa^{0,5}$ und $H_h$ im Bereich von 2,5 bis 5,0 $MPa^{0,5}$ auf.

Die Löslichkeitsparameter nach Hansen können nach dem Programm Hansen Solubility Parameters in Practice (HSPiP) (2. Auflage) wie bei Hanson und Abbot et al. erhältlich bestimmt werden.

Beispiele brauchbarer polymerer Bindemittel werden in Tabelle 1 der WO 2011/076325 A1 offenbart.

Gemäß einer bevorzugten Ausführungsform der vorliegenden Erfindung handelt es sich bei dem inerten Bindemittel um ein Polymer mit einer Glasübergangstemperatur im Bereich von -70 bis 160°C, vorzugsweise 0 bis 150°C, besonders bevorzugt 50 bis 140°C und insbesondere bevorzugt 70 bis 130°C. Die Glasübergangstemperatur lässt sich durch Messen der DSC des Polymers (DIN EN ISO 11357, Erhitzungsgeschwindigkeit 10°C pro Minute) bestimmen.

Die Formulierung gemäß der vorliegenden Erfindung kann zusätzlich eine oder mehrere weitere Komponenten enthalten, wie beispielsweise oberflächenaktive Verbindungen, Gleitmittel, Netzmittel, Dispergiermittel, Hydrophobiermittel, Haftmittel, Fließverbesserer, Entschäumer, Entgasungsmittel, Verdünnungsmittel, die reaktiv oder nicht reaktiv sein können, Hilfsstoffe, Farbmittel, Farbstoffe oder Pigmente, Sensibilisierer, Stabilisatoren, Nanopartikel oder Inhibitoren. Diese weiteren Komponenten sollten jedoch nicht oxidierend oder anders in der Lage sein, chemisch mit dem organischen Halbleitermaterial zu reagieren oder einen elektrisch dotierenden Effekt auf das organische Halbleitermaterial auszuüben.

Überraschende Verbesserungen lassen sich mit flüchtigen Netzmitteln erzielen. Der Ausdruck "flüchtig", wie

er vor- und nachstehend verwendet wird, bedeutet, dass das Mittel aus dem/den organischen Halbleitermaterial(ien) durch Verdampfen entfernt werden kann, nachdem diese(s) Material(ien) auf ein Substrat einer OLED-Vorrichtung unter Bedingungen (wie Temperatur und/oder verminderter Druck), die diese Materialien oder die OLED-Vorrichtung nicht wesentlich beschädigen, abgeschieden wurden. Das bedeutet vorzugsweise, dass das Netzmittel bei dem verwendeten Druck, sehr bevorzugt bei Atmosphärendruck (1013 hPa) vorzugsweise einen Siedepunkt oder eine Sublimationstemperatur von < 350°C, besonders bevorzugt ≤ 300°C, insbesondere bevorzugt ≤ 250°C besitzt. Die Verdampfung kann auch beschleunigt werden, z.B. durch Einsatz von Wärme und/oder vermindertem Druck.

Überraschende Wirkungen lassen sich mit Formulierungen erreichen, die flüchtige Komponenten mit ähnlichen Siedepunkten enthalten. Vorzugsweise liegt der Unterschied des Siedepunktes des Netzmittels und des organischen Lösungsmittels im Bereich von -50°C bis 50°C, besonders bevorzugt im Bereich von -30°C bis 30°C und insbesondere bevorzugt im Bereich von -20°C bis 20°C.

Bevorzugte Netzmittel sind nichtaromatische Verbindungen. Weiteren Vorzug haben nichtionische Verbindungen als Netzmittel. Besonders brauchbare Netzmittel weisen eine Oberflächenspannung von höchstens 35 mN/m, vorzugsweise von höchstens 30 mN/m und besonders bevorzugt von höchstens 25 mN/m auf. Die Oberflächenspannung kann mit einem Kontaktwinkel-Goniometer FTA (First Ten Angstrom) 125 bei 25°C gemessen werden. Einzelheiten zum Verfahren sind bei First Ten Angstrom, wie von Roger P. Woodward, Ph.D., "Surface Tension Measurements Using the Drop Shape Method" veröffentlicht, erhältlich. Vorzugsweise kann die Pendant-Drop-Methode zur Bestimmung der Oberflächenspannung verwendet werden.

Gemäß einem besonderen Aspekt der vorliegenden Erfindung beträgt der Unterschied der Oberflächenspannung des organischen Lösungsmittels und des Netzmittels bevorzugt mindestens 1 mN/m, vorzugsweise mindestens 5 mN/m und besonders bevorzugt mindestens 10 mN/m.

Gemäß einem besonderen Aspekt der vorliegenden Erfindung kann das Netzmittel eine relative Verdampfungsgeschwindigkeit (Butylacetat = 100) von mindestens 0,01, bevorzugt von mindestens 0,1, vorzugsweise von mindestens 0,5, besonders bevorzugt von mindestens 5, ganz besonders bevorzugt von mindestens 10 und insbesondere bevorzugt von mindestens 20 aufweisen.

Unerwartete Verbesserungen lassen sich mit Zusammensetzungen erzielen, die Lösungsmittel und Netzmittel mit einer ähnlichen relativen Verdampfungsgeschwindigkeit (Butylacetat = 100) enthalten. Vorzugsweise liegt der Unterschied der relativen Verdampfungsgeschwindigkeit (Butylacetat = 100) des Netzmittels und des organischen Lösungsmittels im Bereich von -20 bis 20, besonders bevorzugt im Bereich von -10 bis 10. Gemäß einer bevorzugten Ausführungsform der vorliegenden Erfindung kann das Verhältnis der relativen Verdampfungsgeschwindigkeit (Butylacetat = 100) des Netzmittels zur relativen Verdampfungsgeschwindigkeit (Butylacetat = 100) des organischen Lösungsmittels im Bereich von 230:1 bis 1:230, vorzugsweise von 20:1 bis 1:20 und besonders bevorzugt von 5:1 bis 1:5 liegen.

Unerwartete Verbesserungen lassen sich durch Netzmittel erzielen, die ein Molekulargewicht von mindestens 100 g/mol, vorzugsweise mindestens 150 g/mol, besonders bevorzugt mindestens 180 g/mol und insbesondere bevorzugt mindestens 200 g/mol aufweisen.

Geeignete und bevorzugte Netzmittel, die nicht oxidieren oder auf andere Weise chemisch mit den OSC-Materialien reagieren, sind aus der Gruppe bestehend aus Siloxanen, Alkanen, Aminen, Alkenen, Alkinen, Alkoholen und/oder halogenierten Derivaten dieser Verbindungen ausgewählt. Des weiteren können Fluorether, Fluorester und/oder Fluorketone verwendet werden. Besonders bevorzugt sind diese Verbindungen aus Methylsiloxanen mit 6 bis 20 Kohlenstoffatomen, insbesondere 8 bis 16 Kohlenstoffatomen; $C_7$-$C_{14}$ Alkanen, $C_7$-$C_{14}$ Alkenen, $C_7$-$C_{14}$ Alkinen, Alkoholen mit 7 bis 14 Kohlenstoffatomen, Fluorethern mit 7 bis 14 Kohlenstoffatomen, Fluorestern mit 7 bis 14 Kohlenstoffatomen und Fluorketonen mit 7 bis 14 Kohlenstoffatomen ausgewählt. Insbesondere bevorzugte Netzmittel sind Methylsiloxane mit 8 bis 14 Kohlenstoffatomen.

Beispiele von Verbindungen, die als Netzmittel brauchbar und bevorzugt sind, sind in der in WO 2011/076325 A1 offenbart.

Bevorzugt enthält die Formulierung vorzugsweise höchstens 5 Gew.-%, besonders bevorzugt höchstens 3 Gew.-% und insbesondere bevorzugt höchstens 1 Gew.-% an benetzenden Hilfsstoffen. Bevorzugt enthält die Zusammensetzung 0,01 bis 5 Gew.-%, besonders bevorzugt 0,05 bis 3 Gew.-% und insbesondere bevorzugt 0,1 bis 1 Gew.-% Netzmittel.

Die erfindungsgemäße Formulierung kann weitere Verbindungen enthalten. Die weiteren Verbindungen können anorganische oder organische Verbindungen sein. Bevorzugt handelt es sich bei den weiteren Verbindungen um organische Halbleitermaterialien, die ganz bevorzugt ausgewählt werden aus der Gruppe bestehend aus den fluoreszierenden Emittern, phosphoreszierenden Emittern, Host Materialien, MatrixMaterialien, Elektronentransportmaterialien, Elektroneninjektionsmaterialien, Lochleitermaterialien, Lochinjektionsmaterialien, n-Dotanden, Wide-Band-Gap-Materialien, Elektronenblockiermaterialien und Lochblockiermaterialien.

Fluoresziernde Emitter (auch fluoreszierende Dotanden genannt) im Sinne der vorliegenden Erfindung sind Verbindungen, bei denen die Lichtemission durch einen spin-erlaubten Übergang erfolgt, vorzugsweise handelt es sich hierbei um einen Übergang aus einem angeregten Singulett-Zustand.

Vom Begriff phosphoreszierende Emitter (auch phosphoreszierende Dotanden genannt) sind typischerweise Verbindungen umfasst, bei denen die Lichtemission durch einen spin-verbotenen Übergang erfolgt, beispielsweise einen Übergang aus einem Triplett-Zustand oder einem Zustand mit einer höheren Spinquantenzahl, beispielsweise einem Quintett-Zustand, vorzugsweise handelt es sich bei dem Übergang um einen Übergang aus einem Triplett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen.

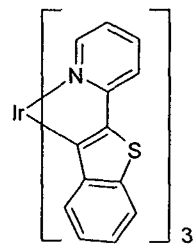
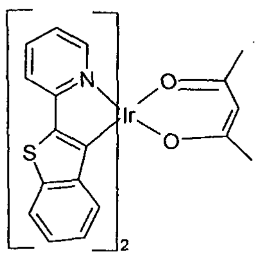
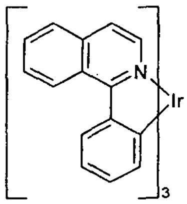

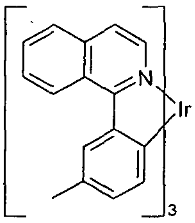
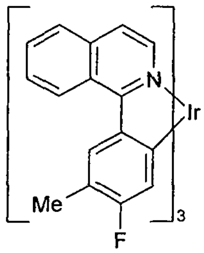
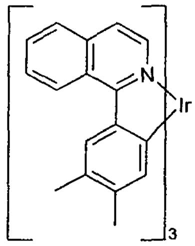

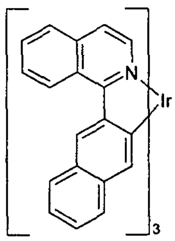
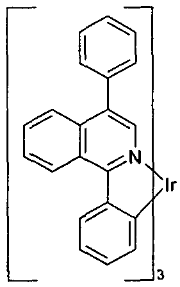
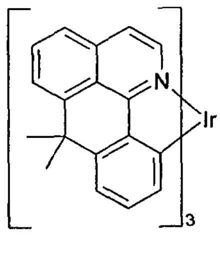

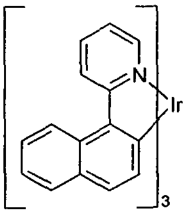
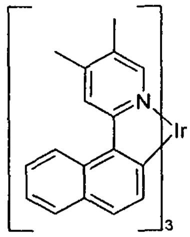
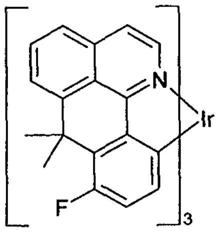

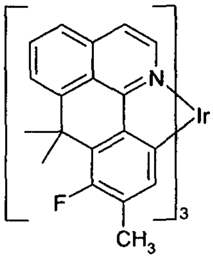
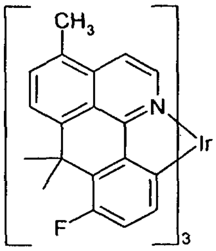
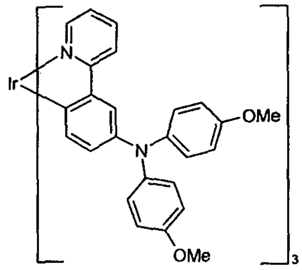
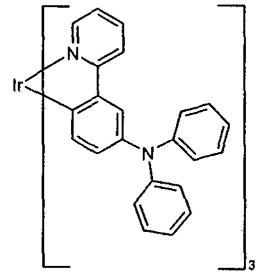
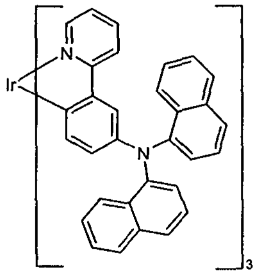
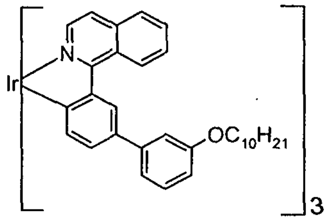
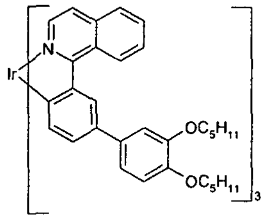
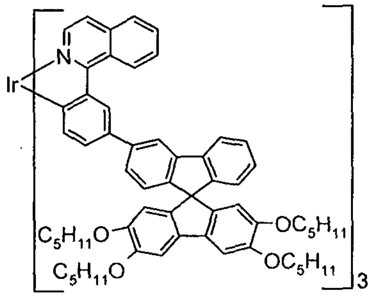
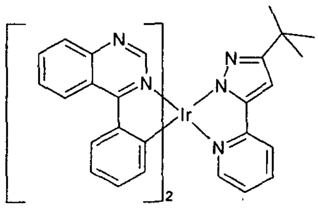
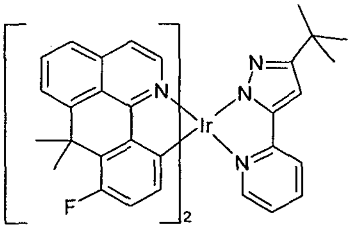
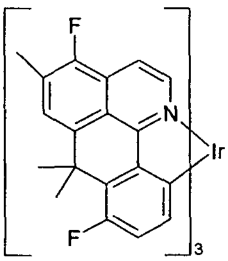
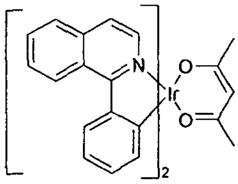
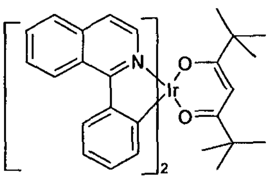
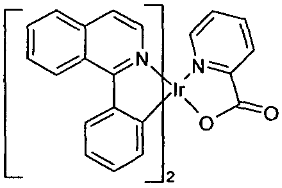
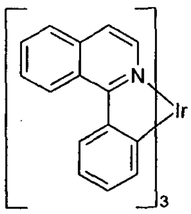
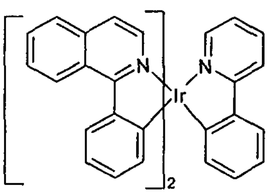
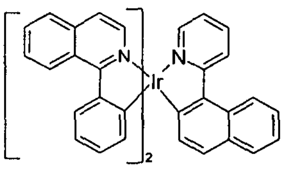
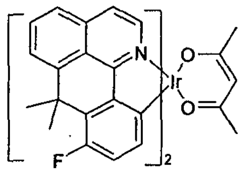

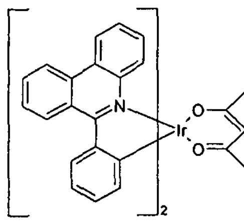
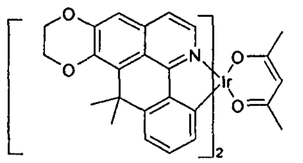
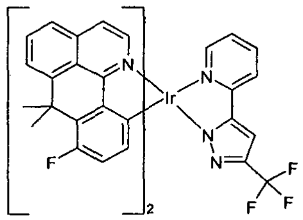

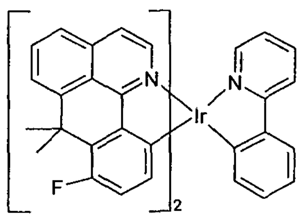
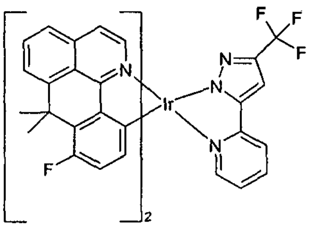
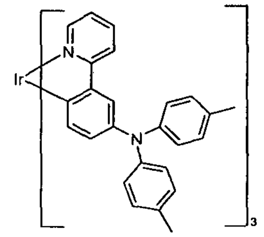

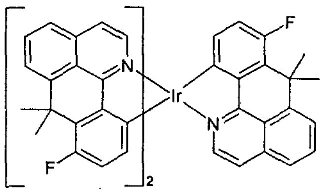
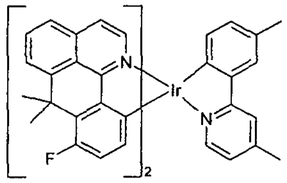
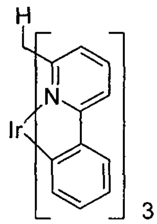

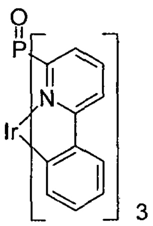
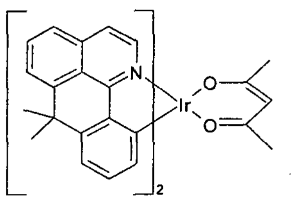
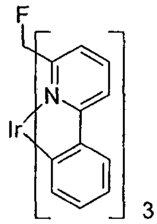

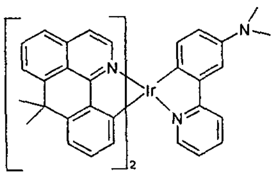
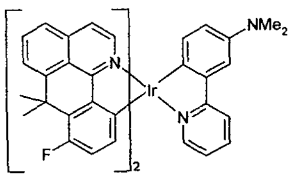
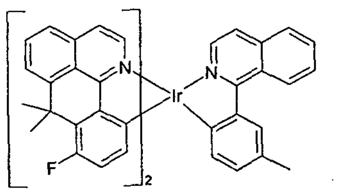

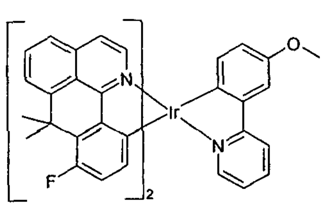
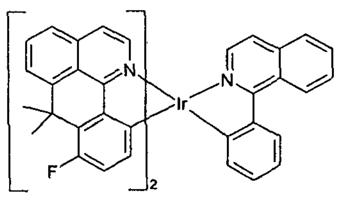
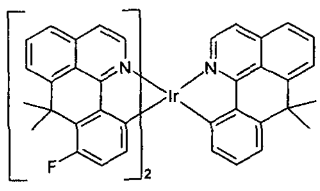

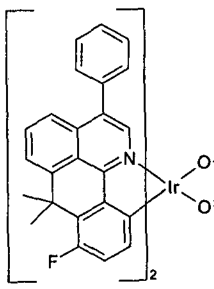
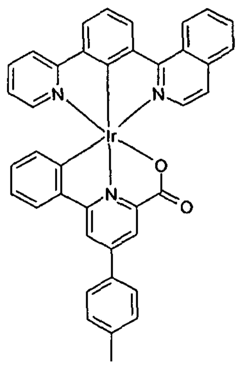
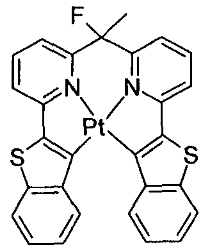

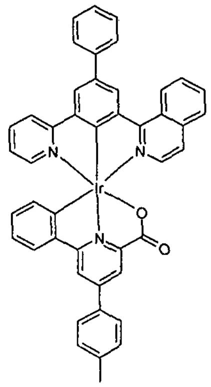
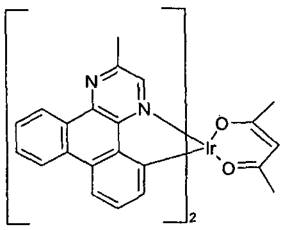
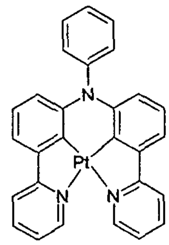

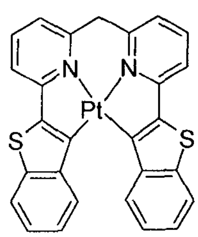
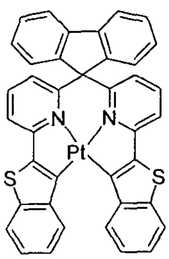
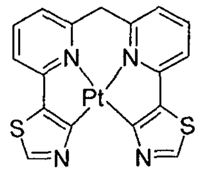

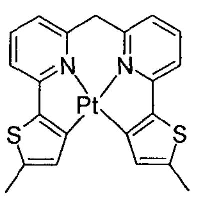
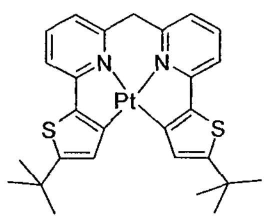
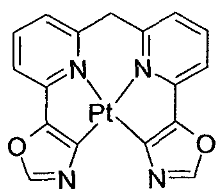

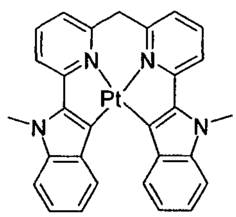
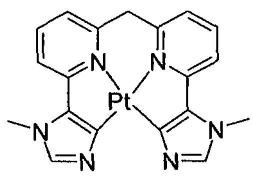
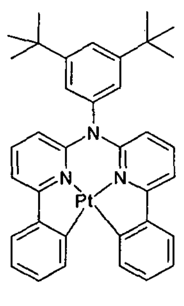

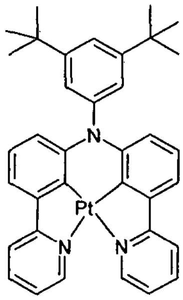
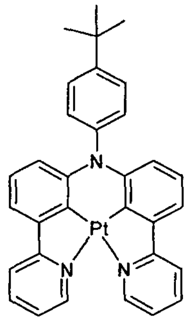
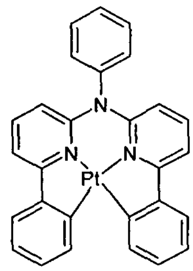

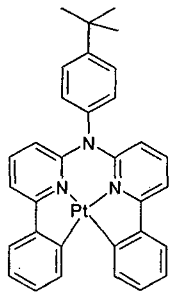
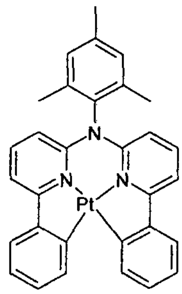
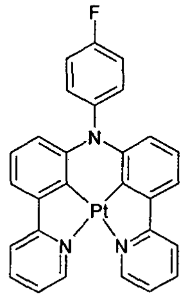

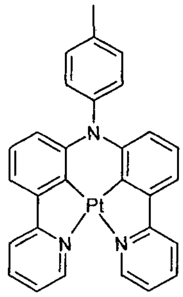
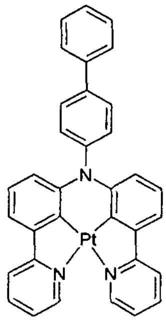
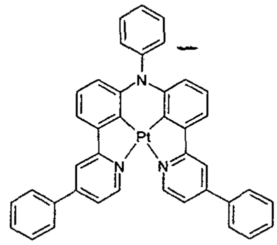

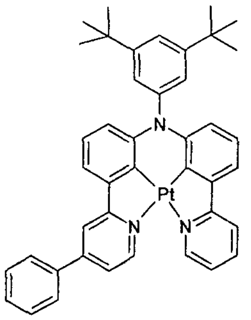
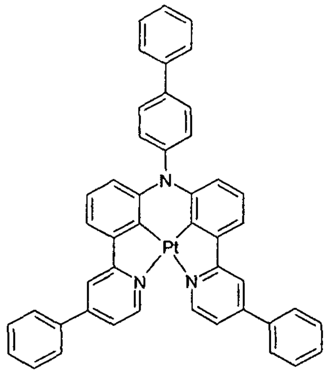
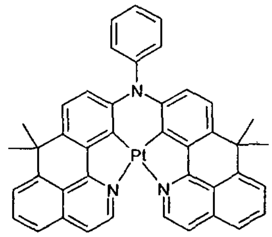

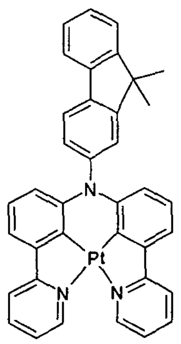
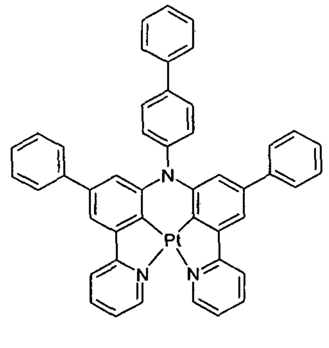
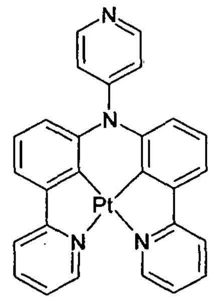

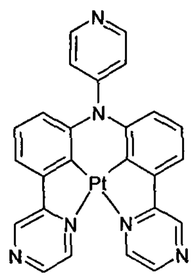
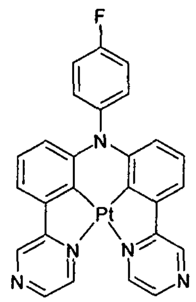
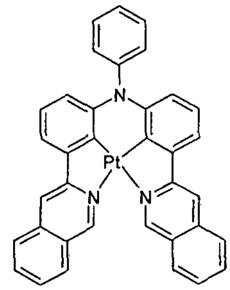

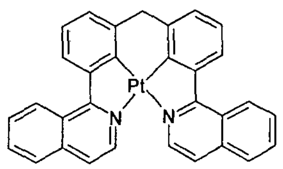
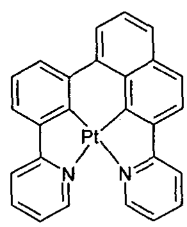
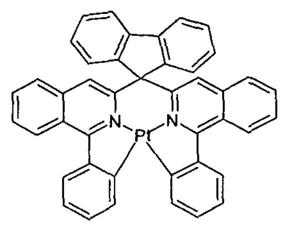

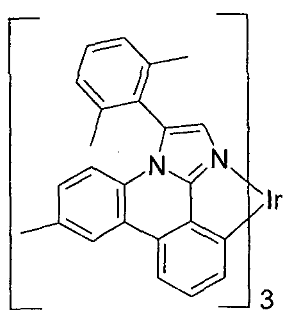
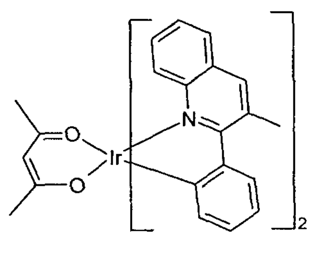
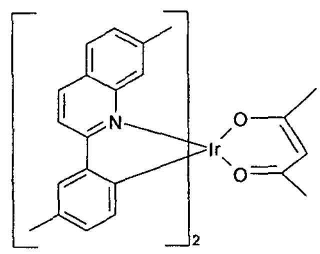

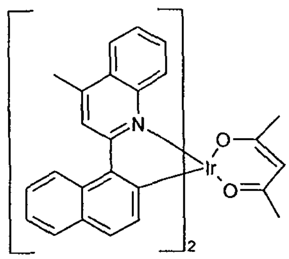
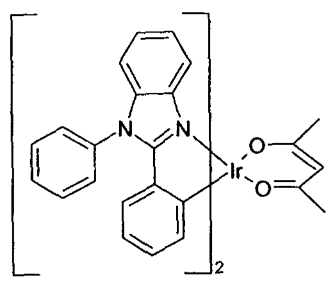
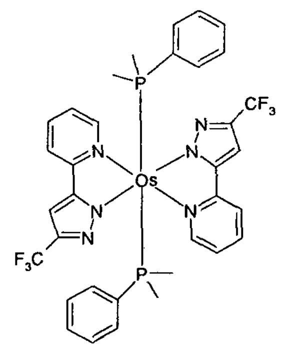

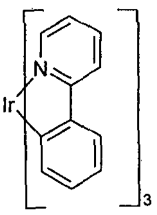
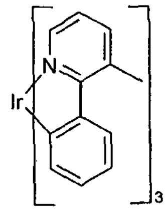
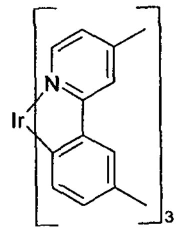

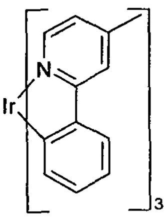
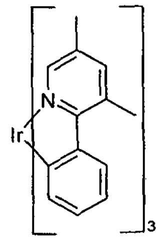
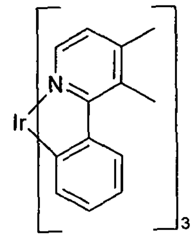

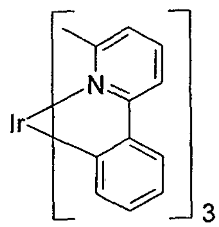
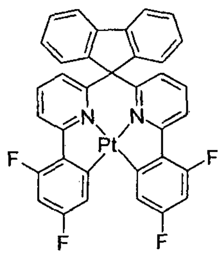
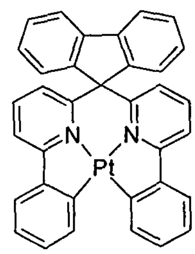

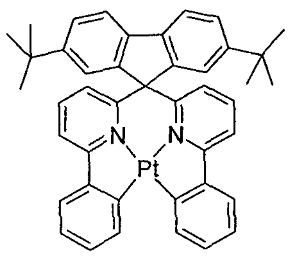
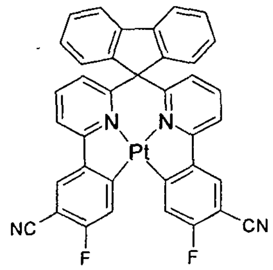
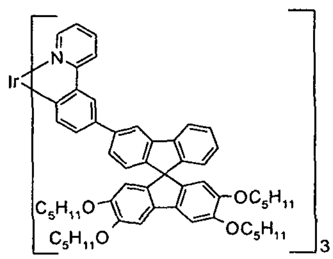

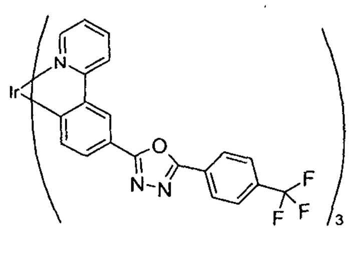
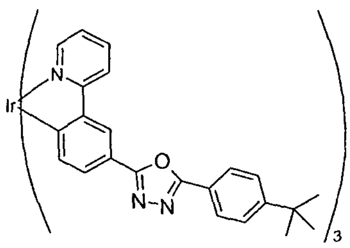
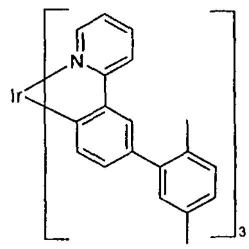

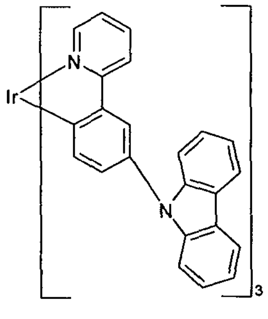
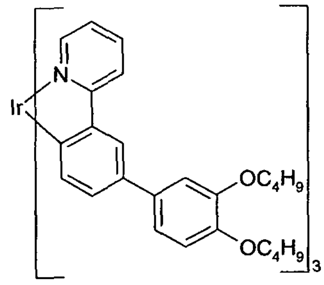
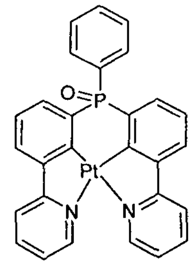

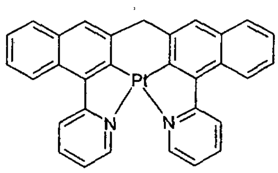

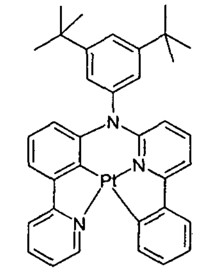

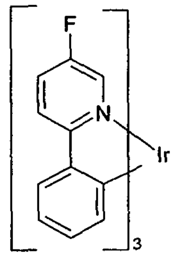

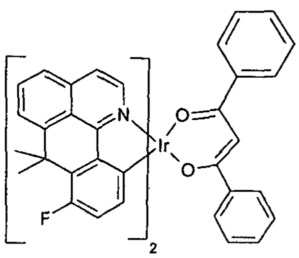

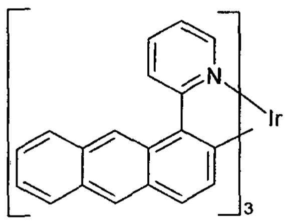

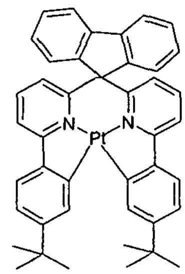

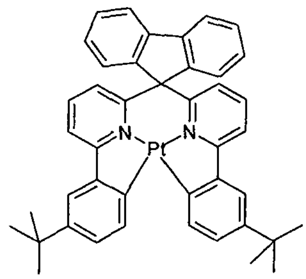

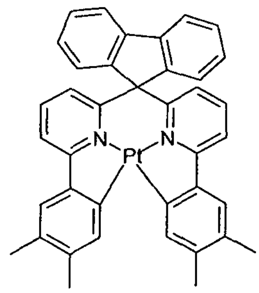

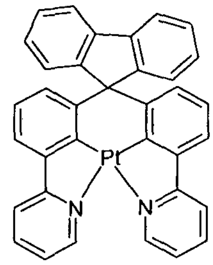

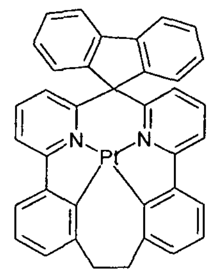

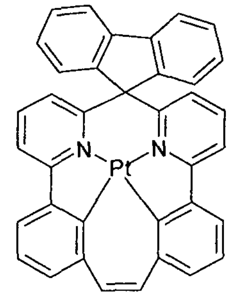

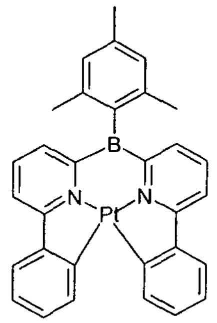

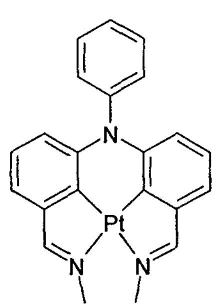

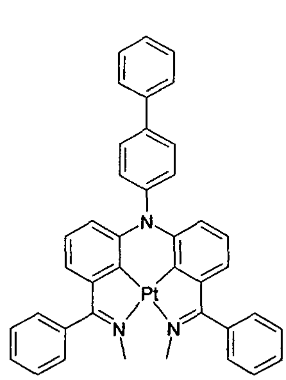

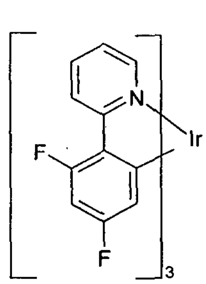

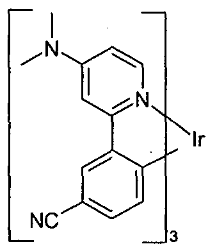 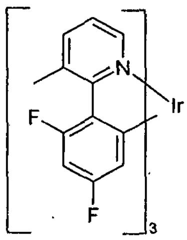 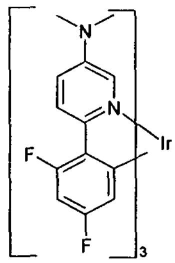

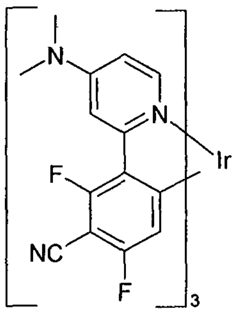 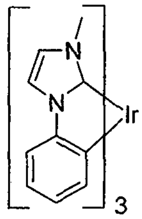 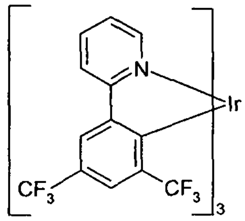

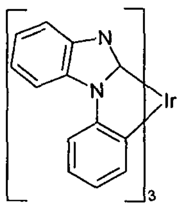 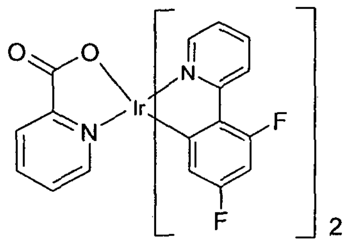 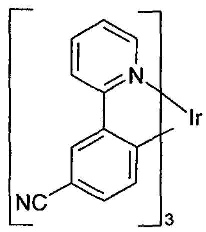

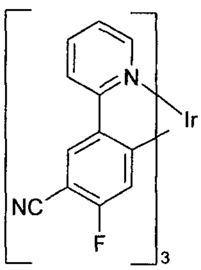 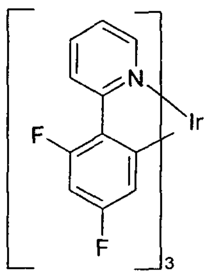 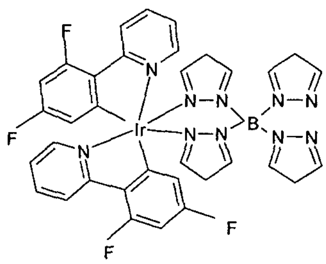

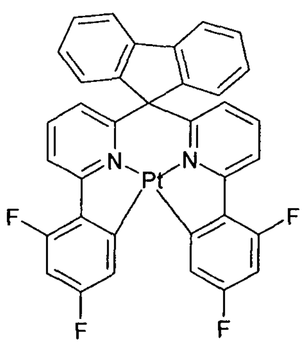 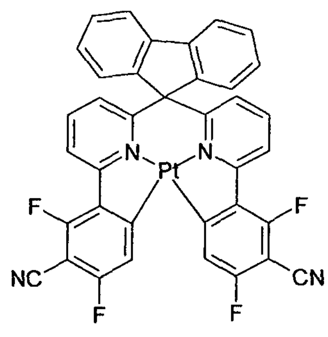 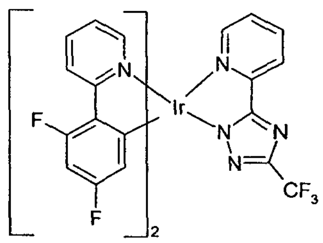

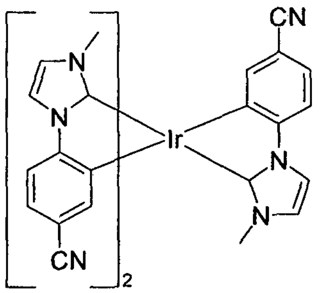
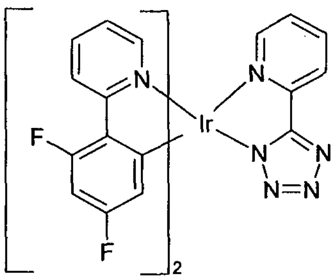
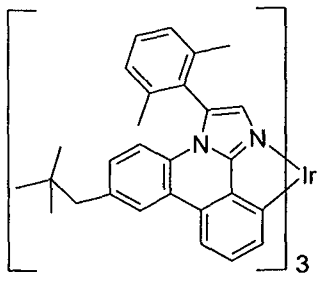

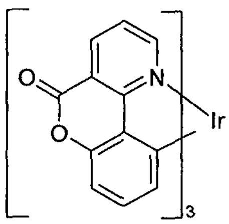
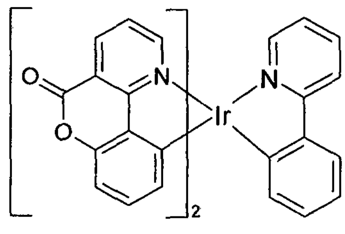
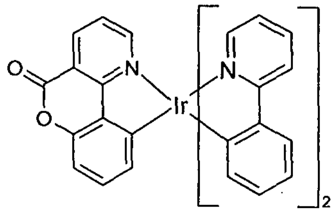

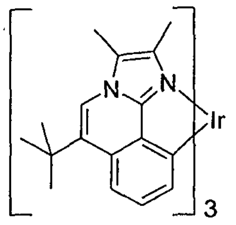
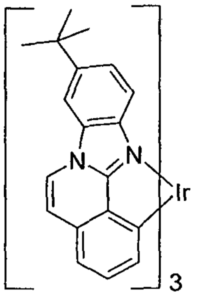
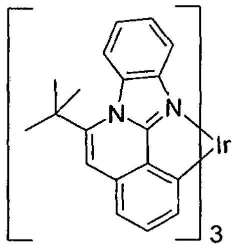

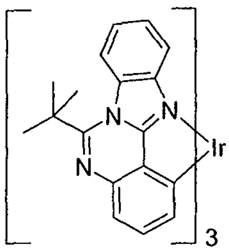
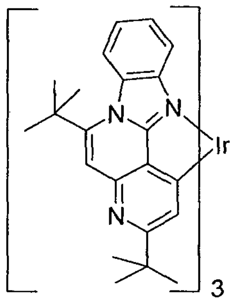
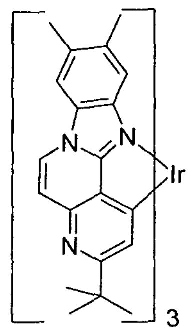

Unter einem Dotanden im Allgemeinen wird vorliegend in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Die oben genannten weiteren Verbindungen, die in den erfindungsgemäßen Formulierungen enthalten sein können sind dem Fachmann gut bekannt. Der Fachmann kann daher aus einem großen Repertoire bekannter Verbindungen auswählen.

In einer bevorzugten Ausführungsform enthält die Formulierung als weitere Verbindung wenigstens ein organisches Matrixmaterial, wobei es sich bei dem organischen Matrixmaterialien bevorzugt um Carbazole, Indenocarbazole, Indolocarbazole (z.B. EP 13001800.5), Triazine, Pyrimidine, Lactame, Ketone, Phosphinoxide, wie beispielsweise in EP 13001797.3 offenbart, Triphenylene, Diarylfluorene (insbesondere 9,9-Diarylfluorene wie in WO 2009/124627 offenbart) oder Dibenzofurane handelt.

Ganz bevorzugt ist, wenn es sich bei dem Matrixmaterial um ein lochleitendes Matrixmaterial handelt, das vorzugsweise aus der Gruppe der Carbazole, Indenocarbazole, Indolocarbazole ausgewählt wird.

Weiterhin bevorzugt ist, wenn es sich bei dem Matrixmaterial um ein elektronenleitendes Matrixmaterial handelt,

das vorzugsweise aus der Gruppe der Triazine, Pyrimidine, Lactame, Ketone und Phosphinoxide ausgewählt wird.

Ferner bevorzugt ist, wenn die Formulierung eine Triphenylen-Matrix enthält. Typische und im Sinne der vorliegenden Erfindung bevorzugte Triphenylen-Matrixverbindungen sind in EP 1888708 offenbart.

Auch bevorzugt ist, wenn die Formulierung eine Dibenzofuran-Matrix enthält.

Ganz bevorzugt ist, wenn das organische Matrixmaterial, das in der Formulierung enthalten ist und die TADF Verbindung die folgenden Bedingung erfüllen.

[LUMO(TADF) - HOMO(Matrix)] ist größer oder gleich [$S_1$(TADF) - 0.4 eV],

wobei $S_1$(TADF) der erste angeregte Singulett-Zustand $S_1$ der TADF-Verbindung ist. LUMO(TADF) ist die LUMO-Energie der TADF-Verbindung und HOMO(Matrix) ist die HOMO-Energie der Matrix.

Ganz besonders bevorzugt ist, wenn die Formulierung neben einem ersten Matrixmaterial auch ein zweites Matrixmaterial enthält. Mixed-Matrix Systeme zeigen besonders vorteilhafte Wirkungen. Elektronische Vorrichtungen enthaltend diese Mixed-Matrix Systeme weisen oft sehr gute Leistungsdaten auf. Mixed-Matrix-Systeme sind, beispielsweise, in US 6,392,250 B1 und in US 6,803,720 B2 offenbart.

Ganz besonders bevorzugt ist, wenn es sich bei dem ersten Matrixmaterial um ein elektronentransportierendes Matrixmaterial und bei dem zweiten Matrixmaterial um ein lochtransportierendes Matrixmaterial handelt; bevorzugt handelt es sich bei dem elektronentransportierenden Matrixmaterial um Triazine, Pyrimidine, Lactame, Ketone und Phosphinoxide; bevorzugt handelt es sich bei den lochtransportierenden Matrixmaterialien um Carbazole, Indenocarbazole, Indolocarbazole.

Weiterhin ganz besonders bevorzugt ist, wenn es sich sowohl bei dem ersten als auch bei dem zweiten Matrixmaterial um elektronentransportierende Matrixmaterialien handelt, die bevorzugt Triazine, Pyrimidine, Lactame, Ketone, Phosphinoxide darstellen.

Auch bevorzugt ist, wenn es sich bei sowohl dem ersten als auch bei dem zweiten Matrixmaterial um lochtransportierende Matrixmaterialien handelt, die bevorzugt Carbazole, Indenocarbazole, Indolocarbazole darstellen.

Insbesondere bevorzugt ist, wenn es sich bei dem ersten Matrixmaterial entweder um ein elektronentransportierendes oder um ein lochtransportierendes Matrixmaterial handelt und dass es sich bei dem zweiten Matrixmaterial um eine Verbindung handelt, die eine große Bandlücke (band gap) aufweisen. Diese Verbindungen werden auch Wide-Band-Gap Materialien genannt (US 7,294,849).

Vorzugsweise hat das Wide-Band-Gap Material eine Bandlücke von 2.5 eV oder mehr, bevorzugt 3.0 eV oder mehr, ganz bevorzugt von 3.2 eV oder mehr aufweisen, ganz besonders bevorzugt von 3.5 eV oder mehr und insbesondere bevorzugt von 3.7 eV oder mehr. Die Bandlücke wird vorliegend aus der Differenz der Energieniveaus des HOMOs und des LUMOs berechnet.

Die TADF-Verbindung kann als Emitter in elektronischen Vorrichtungen eingesetzt werden. In einer weiteren Ausführungsform der vorliegenden Erfindung kann die TADF-Verbindung auch als Matrixmaterial verwendet werden. In diesem Fall überträgt die TADF-Verbindung seine Energie auf einen Emitter, der letztlich Strahlung aus der elektronischen Vorrichtung emittiert. Hierfür eignet sich grundsätzlich jeder Emitter. Die Verwendung von TADF-Verbindungen als Matrix ermöglicht elektronische Vorrichtungen mit besonders vorteihaften Leistungsdaten. Besonders bevorzugt ist, wenn die Emitter ein Nanoteilchen ist.

Es ist daher sehr vorteilhaft, wenn die Formulierung nanokristalline Verbindungen oder Nanoteilchen enthält. Zu den Nanokristallen und Nanoteilchen zählen die Quantenpunkte (quantum rods) und Quantenstäbchen (quantum rods). Mit Hilfe dieser Verbindungen lassen sich effiziente OLEDs und andere organische elektronische Vorrichtungen kostengünstig herstellen, die eine sehr geringe Ausfallrate aufweisen und eine Emissionen mit schmalen Banden zeigen. Weiterhin lassen sich emittierende Vorrichtungen erhalten, die ausgezeichnete Farbreinheiten und Farbqualitäten (CRI - color rendering index) zeigen. Weiterhin kann die Emissionsfarbe (CIE 1931 RGB) solcher OLEDs sehr leicht und genau eingestellt werden.

Ganz besonders bevorzugt ist, wenn die Formulierung neben Nanoteilchen und TADF-Verbindung(en) noch weitere Matrixverbindungen enthält, wobei es sich bevorzugt um die oben genannten elektronentransportierenden Matrixverbindungen oder um die oben genannten lochtransportierneden Matrixverbindungen handelt.

Sowohl Quantenpunkte als auch Quantenstäbchen können sehr einfach hergestellt werden. Die Größe der Teilchen, die bestimmender Faktor für die Farbe der emittierten Strahlung darstellt, ist leicht einzustellen. Weiterhin sind die Quantenpunkte und Quantenstäbchen löslich in vielen gängigen Lösungsmitteln und eignen sich sehr gut für lösungsbasierte Herstellprozesse.

Die ersten monodispersen kolloidalen Quantenpunkte enthaltend ein halbleitendes Material basierten auf CdE (E = S, Se, Te) und wurden mit Hilfe der sogenannten TOPO (Trioctylphosphinoxid) Methode hergestellt (J. Am. Chem. Soc. 115[19], 8706-8715, 1993).

Dem Fachmann ist eine Vielzahl von Methoden zur Herstellung von Quantenpunkten und Quantenstäbchen bekannt, wobei bevorzugt lösungsbasierte Methoden kolloidaler Systeme zum kontrollierten Wachstum anorganischer Quantenpunkte eingesetzt werden (Science 271:933 (1996); J. Am. Chem. Soc. 30:7019-7029 (1997); J. Am. Chem. Soc. 115:8706 (1993)).

Geeignete Halbleiter, die in Quantenpunkten und Quantenstäbchen verwendet werden sind ausgewählt aus der der Gruppe bestehend aus

Elementen der Gruppen II bis VI, so wie Z.B. CdSe, CdS, CdTe, ZnSe, ZnO, ZnS, ZnTe, HgS, HgSe, HgTe und Mischungen davon, wie z.B. CdZnSe;

Elementen der Gruppe III bis V, wie z.B. InAs, InP, GaAs, GaP, InN, GaN, InSb, GaSb, AlP, AlAs, AlSb und Mischungen davon, wie z.B. InAsP, CdSeTe, ZnCdSe, InGaAs;

Elementen der Gruppe IV bis VI, wie z.B. PbSe, PbTe and PbS und Mischungen davon;

Elementen der Gruppe III bis VI, wie z.B. InSe, InTe, InS, GaSe und Mischungen davon, wie z.B. InGaSe, InSeS;

Elementen der Gruppe IV Halbleiter, wie z.B. Si and Ge und Mischungen davon, sowie Mischungen der zuvor genannten Materialien.

Weiter geeignete Halbleiter für Quantenpunkte und Quantenstäbchen umfassen solche, die in US10/796,832 offenbart sind und umfassen jede Art von Halbleitern umfassend Elemente der Gruppen II bis VI, III bis V, IV bis VI und IV Halbleiter. Eine Auswahl besonders geeigneter Halbleiter ist Si, Ge, Sn, Se, Te, B, C (auch Diamand), P, BN, BP, BAs, AlN, AlP, AlAs, AlS, AlSb, BaS, BaSe, BaTe, CaS, CaSe, CaTe, GaN, GaP, GaAs, GaSb, InN, InP, InAs, InSb, AlN, AlP, AlAs, AlSb, GaN, GaP, GaAs, GaSb, ZnO, ZnS, ZnSe, ZnTe, CdS, CdSe, CdTe, HgS, HgSe, HgTe, BeS, BeSe, BeTe, MgS, MgSe, GeS, GeSe, GeTe, SnS, SnSe, SnTe, PbO, PbS, PbSe, PbTe, CuF, CuCl, CuBr, CuI, $Si_3N_4$, $Ge_3N_4$, $Al_2O_3$, $(Al, Ga, In)_2$ $(S, Se, Te)_3$, $Al_2CO$, und eine geeignete Kombination von zwei oder mehr der genannten Halbleiter..

Es ist bevorzug, wenn die Quantenpunkte oder Quantenstäbchen ausgewählt werden aus Elementen der Gruppen II-VI, III-V, IV-VI und IV Halbleitern, ganz bevorzugt aus ZnO, ZnS, ZnSe, ZnTe, CdS, CdSe, CdTe, HgS, HgSe, HgTe, MgS, MgSe, GeS, GeSe, GeTe, SnS, SnSe, SnTe, PbO, PbS, PbSe, PbTe, GaN, GaP, GaAs, GaSb, InN, InP, InAs, InSb, AlN, AlP, AlAs, AlSb, GaN, GaP, GaAs, GaSb, und einer Kombination aus diesen.

In QDs and QRs, photoluminescence and electroluminescence arise from the band edge states of the nanoparticle. The radiative band-edge emission from nanoparticles competes with non-radiative decay channel originating from surface electronic states, as reported by X. Peng, et al., J. Am. Chem. Soc. Vol119:7019-7029 (1997).

Als besonders vorteilhaft haben sich auch Quantenpunkte und Quantenstäbchen gezeigt, die eine Core-Shell Struktur aufweisen (J. Am. Chem. Soc. Vol119:7019-7029 (1997).

Core-Shell Strukturen können erhalten werden, wenn organometallische Vorstufen, welche die Shell Materialien enthalten, als Vorstufen verwendet werden. Diese Vorstufen werde zu einer Reaktionsmischung mit dem Core-Nanoteilchen gegeben. Weitere Einzelheiten zum Herstellungsverfahren sind dem Fachmann aus dem Stand der Technik gut bekannt.

In einer bevorzugten Ausführungsform wird ZnS als Shell-Material verwendet.

Weiter bevorzugt ist, wenn die Quantenpunkte und Quantenstäbchen halbleitende Materialien der Gruppe II-VI, Mischungen davon und Core-Shell Strukturen davon darstellen. Ganz bevorzugt sind dabei CdSe, CdS, CdTe, ZnSe, ZnS, ZnTe, Mischungen davon.

Die Quantenpunkte und Quantenstäbchen können weitere Liganden enthalten, die an die Oberfläche der Teilchen gebunden sind. Geeignete Liganden hierfür sind im Stand der Technik gut bekannt und sind, beispielsweise, offenbart in US 10/656910 und US 60/578236. Hierdurch können verschiedene Eigenschaften der Quantenpunkte und Quantenstäbchen verbessert werden. Hierdurch kann die Löslichkeit in bestimmten Lösungsmittel Matrixmaterialien und Polymeren verbessert werden. Weitere bevorzugte Liganden sind in US 2007/0034833A1 und US 20050109989A1 offenbart.

Die hierin verwendeten Begriffe Quantenpunkt und Quantenstäbchen stehen für Nanoteilchen, die im Wesentlichen eine monodisperse Größenverteilung aufweisen. Die Dimension der Teilchen beträgt 500 nm und weniger, bis hin zu einer Dimension von ca. 1 nm. Monodispers bedeutet, dass die Teilchen eine Größenverteilung von +-10% der genannten Dimension der Teilchen aufweist.

Typische Strukturen und Verfahren zur Herstellung von Quantenstäbchen sind in US 2013/0135558 A1 offenbart.

Die erfindungsgemäßen Formulierungen können zur Herstellung von Schichten in elektronischen Vorrichtungen verwendet werden, wobei die Schichten, die mittels der erfindungsgemäßen Formulierung hergestellt wird aus Lösung aufgebracht wird.

Bei der Herstellung organischer elektronischer Vorrichtungen, wie z.B. den OLEDs, wird zwischen zwei grundsätzlich verschiedenen Verfahren unterscheiden. Beim ersten Verfahren werden die relevanten Verbindungen im Vakuum aufgedampft. Dieses Verfahren ist sehr aufwendig und kostenintensiv. Bei dem zweiten Verfahren werden die relevanten Verbindungen aus der Lösung aufgebracht.

Bestimmte elektronischen Vorrichtungen sind aus Mehrschichtsystemen aufgebaut. Bei der Herstellung solcher Mehrschichtsysteme können beide oben genannte Verfahren eingesetzt werden. Häufig werden einzelne Schichten aufgedampft, während andere Schichten aus Lösung prozessiert werden.

Ein weiterer Gegenstand der vorliegenden Erfindung ist daher ein Verfahren zur Herstellung einer organischen

elektronischen Vorrichtung, dadurch gekennzeichnet, dass wenigstens eine Schicht der elektronischen Vorrichtung mit Hilfe der erfindungsgemäßen Formulierung aus Lösung hergestellt wird.

Typische Verfahren zur Herstellung von Schichten aus der Lösung sind z. B. das Spincoating, oder ein beliebiges Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck). Hierfür sind Formulierungen erforderlich.

Bei den organischen elektronischen Vorrichtungen handelt es sich bevorzugt um eine organische integrierte Schaltung (OIC), einen organischen Feld-Effekt-Transistor (OFET), einen organischen Dünnfilmtransistor (OTFT), eine organisch Elektrolumineszenzvorrichtung, eine organische Solarzelle (OSC), einen organischen optischen Detektor, einen organischen Photorezeptor, bevorzugt aber um eine organische Elektroluminesszenzvorrichtung.

Ganz bevorzugte organische elektrolumineszierende Vorrichtungen sind die organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

Die Formulierungen können mit Hilfe von Verfahren hergestellt werden die dem Fachmann gut geläufig sind. Typischerweise werden die einzelnen Komponenten der Formulierung gemischt und gerührt, ggf. auch unter Zuführ von Wärme. Häufig wird die Formulierung auch entgast oder mit Inertgasen übersättigten Lösungsmitteln hergestellt. Insgesamt ist darauf zu achten, dass nur Lösungsmittel und andere Komponenten sehr hoher Reinheit verwendet werden, um eine Kontamination der elektronischen Vorrichtungen mit schädlichen Verbindungen zu vermeiden. Insbesondere ist darauf zu achten, dass der Wasser-, Sauerstoff und Halogengehalt in der Formulierung niedrig gehalten wird, da insbesondere die Leistungsdaten organischer Elektrolumineszenzvorrichtungen durch deren Anwesenheit stark beeinträchtigt werden können.

Die erfindungsgemäßen Formulierungen zeichnen sich durch einen oder mehrere der folgenden überraschenden Vorteile gegenüber dem Stand der Technik aus:

1. Organische elektronische Vorrichtungen, insbesondere organische Elektrolumineszenzvorrichtungen hergestellt mit Hilfe der erfindungsgemäßen Formulierungen weisen sehr gute Stabilitäten auf. Insbesondere kann mit Hilfe der Formulierungen die Anzahl von Ausfällen signifikant reduziert werden gegenüber elektronischen Vorrichtungen, die mit Hilfe von Vakuumverdampfen hergestellt wurden.
2. Organische elektronische Vorrichtungen, insbesondere organische Elektrolumineszenzvorrichtungen hergestellt mit Hilfe der erfindungsgemäßen Formulierungen weisen vergleichbare Effizienzen und Spannungen auf im Vergleich zu Vorrichtungen aus dem Stand der Technik.
3. Organische elektronische Vorrichtungen, insbesondere organische Elektrolumineszenzvorrichtungen hergestellt mit Hilfe der erfindungsgemäßen Formulierungen sind leicht und kostengünstig herzustellen und eignen sich daher besonders gut für die Massenproduktion kommerzieller Produkte.
4. Mit Hilfe der erfindungsgemäßen Formulierungen können auf sehr einfache und kostengünstige Weise Schichten organischer elektronischer Vorrichtungen, insbesondere in organischen Elektrolumineszenzvorrichtungen hergestellt werden, die mehrere Komponenten (beispielsweise mehrere Matrixverbindungen) enthalten.
5. Mit Hilfe der erfindungsgemäßen Formulierungen können die Herstellverfahren sehr leicht an neue Anforderungen angepasst werden.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße organische Elektrolumineszenzvorrichtungen herstellen.

## Beispiele

### Beispiel 1

**Bestimmungsverfahren**

**Bestimmung von HOMO, LUMO, Singulett- und Triplettniveau**

Die Energieniveaus der Molekülorbitale sowie die Energie des niedrigsten Triplettzustands $T_1$ bzw. des niedrigsten angeregten Singulettzustands $S_1$ der Materialien werden über quantenchemische Rechnungen bestimmt. Hierzu wird vorliegend das Programmpaket "Gaussian09, Revision D.01" (Gaussian Inc.) verwendet. Zur Berechnung organischer Substanzen ohne Metalle (mit Methode "org." bezeichnet) wird zuerst eine Geometrieoptimierung mit der semiempirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geometrie eine Energierechnung (single point) für den elektronischen Grundzustand und das Triplett-Niveau. Hierbei wird die TDDFT-Methode (time dependent density functional theory) B3PW91 mit dem Basissatz 6-31G(d) (Gaussian-Eingabezeile "# B3PW91/6-31G(d) td=(50-50,nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt, wie oben beschrieben, analog zu der der organischen Substanzen, mit dem Unterschied, dass für das Metallatom der Basissatz "LanL2DZ" und für die Liganden der Basissatz "6-31 G(d)" verwendet wird (Gaussian-Eingabezeile "#B3PW91/gen pseudo=lanl2 td=(50-50,nstates=4)"). Aus der Energierechnung erhält man z.B. das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Analog erhält man die Energien in Hartree-Einheiten der anderen Energieniveaus wie HOMO-1, HOMO-2,... LUMO+1, LUMO+2 usw..

Im Sinne dieser Anmeldung werden die anhand von CV Messungen kalibrierten Werte ((HEh*27.212)-0.9899)/1.1206 in eV als Energiniveaus der besetzten Orbitale angesehen.

Im Sinne dieser Anmeldung werden die anhand von CV Messungen kalibrierten Werte ((LEh*27.212)-2.0041)/1.385 in eV als Energiniveaus der unbesetzten Orbitale angesehen.

Der niedrigste Triplettzustand $T_1$ eines Materials ist definiert als die relative Anregungsenergie (in eV) des Triplettzustands mit der niedrigsten Energie, der sich aus der quantenchemischen Energierechnung ergibt.

Der niedrigste angeregte Singulettzustand $S_1$ ist definiert als die relative Anregungsenergie (in eV) des Singulettzustands mit der zweitniedrigsten Energie, der sich aus der quantenchemischen Energierechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.). Vorliegend wird zur Berechnung der Energien das Programmpaket "Gaussian09W" verwendet.

In Tabelle 2 sind die HOMO- und LUMO-Energieniveaus sowie $S_1$ und $T_1$ der verschiedenen Materialien angegeben.

**Bestimmung von Molekülorbitalüberlappungen**

Die Überlappung der Molekülorbitale, die bei bestimmten elektronischen Übergängen beteiligt sind (Charge-Transfer-Zustände) wird mit Hilfe des Parameters $\Lambda$ beschrieben. Dabei ist dem Fachmann die Bedeutung des Parameters $\Lambda$ gut bekannt. Die Bestimmung des Parameters mittels Verfahren, die im Stand der Technik beschrieben sind, bereitet dem Fachmann keinerlei Schwierigkeiten. Im Rahmen der vorliegenden Erfindung wird der Parameter $\Lambda$ anhand der PBHT-Methode gemäß D. J. Tozer et al. (J. Chem. Phys. 128, 044118 (2008)) bestimmt, die beispielsweise in dem Programmpaket Q-Chem 4.1 von Q-Chem, Inc. implementiert ist. Dabei werden die Molekülorbitale gemäß dem oben beschriebenen Verfahren berechnet. Anschließend werden die räumlichen Überlappungen für alle möglichen Paare von besetzten Molekülorbitalen, $\varphi_i$, und unbesetzten (virtuellen) Molekülorbitalen, $\varphi_a$, gemäß folgender Gleichung ermittelt

$$O_{ia} = \langle |\varphi_i| | |\varphi_a| \rangle$$

wobei für die Berechnung die Beträge der Orbitale verwendet werden.

Der Parameter Λ ergibt sich dann aus der gewichteten Summe über alle Paare ia von besetzten und unbesetzten Molekülorbitalen gemäß

$$\Lambda = \frac{\sum_{ia} \kappa_{ia}^2 O_{ia}}{\sum_{ia} \kappa_{ia}^2}$$

wobei der Wert von $\kappa_{ia}$ gemäß Tozer et al. aus den Orbitalkoeffizienten in den Anregungsvektoren der gelösten TD-Eigenwertgleichung (Time-Dependent) ermittelt wird und wobei $0 \leq \Lambda \leq 1$ gilt.

**Bestimmung der PL-Quanteneffizienz (PLQE)**

Von den verwendeten Emissionsschichten wird ein 50 nm dicker Film auf ein Quarzsubstrat aufgebracht. Dieser Film enthält dieselben Materialien in denselben Konzentrationen wie in der Emissionsschicht der entsprechenden OLED, es sei denn die Emissionsschicht enthält eine oder mehrere weitere Komponenten (z.B. Quantenpunkte, anorganische Halbleiter oder organisch Halbleiter). In diesem Fall enthält der Film zur Messung der PLQE alle Materialien bis auf die weiteren Komponenten, die Mischungsverhältnisse der enthaltenen Materialien entsprechen denen in der Emissionsschicht der OLED. Bei der Herstellung der Filme zur Messung der PLQE werden die gleichen Herstellungsbedingungen wie bei der Herstellung der Emissionsschicht für die OLEDs verwendet. Von dem Film wird ein Absorptionsspektrum im Wellenlängenbereich von 350-500 nm gemessen. Hierzu wird das Reflexionsspektrum R(λ) sowie das Transmissionsspektrum T(λ) der Probe unter einem Einfallswinkel von 6° (also nahezu senkrechter Einfall) bestimmt. Als Absorptionsspektrum im Sinne dieser Anmeldung wird A(λ)=1-R(λ)-T(λ) definiert.

Gilt A(λ) kleiner oder gleich 0.3 im Bereich 350-500 nm, so wird die zum Maximum des Absorptionsspektrums gehörige Wellenlänge im Bereich 350-500 nm als $\lambda_{exc}$ definiert. Gilt für irgendeine Wellenlänge A(λ) größer als 0.3, so wird als $\lambda_{exc}$ die größte Wellenlänge definiert, bei der A(λ) von einem Wert kleiner 0.3 zu einem Wert größer 0.3 oder von einem Wert größer 0.3 zu einem Wert kleiner 0.3 wechselt.

Zur Bestimmung der PLQE wird ein Messplatz Hamamatsu C9920-02 verwendet. Das Prinzip beruht auf der Anregung der Probe mit Licht definierter Wellenlänge und der Messung der absorbierten und emittierten Strahlung. Die Probe befindet sich während der Messung in einer Ulbrichtkugel ("integrating sphere"). Das Spektrum des Anregungslichts ist in etwa gaußförmig mit einer Halbwertsbreite von kleiner als 10 nm und Peakwellenlänge $\lambda_{exc}$ wie oben definiert.

Die PLQE wird nach dem für den genannten Messplatz üblichen Auswerteverfahren bestimmt. Es ist strengstens darauf zu achten, dass die Probe zu keinem Zeitpunkt mit Sauerstoff in Berührung kommt, da die PLQE von Materialien mit kleinem energetischen Abstand zwischen $S_1$ und $T_1$ durch Sauerstoff sehr stark reduziert wird (H. Uoyama et al., Nature 2012, Vol. 492, 234). Die Messung erfolgt bei Raumtemperatur.

**Bestimmung der Abklingzeit**

Zur Bestimmung der Abklingzeit wird eine Probe verwendet, die wie oben unter "Bestimmung der PL-Quanteneffizienz (PLQE)" beschrieben hergestellt wird. Die Probe wird bei Raumtemperatur durch einen Laserpuls angeregt (Wellenlänge 266 nm, Pulsdauer 1.5 ns, Pulsenergie 200 μJ, Strahldurchmesser 4 mm). Die Probe befindet sich hierbei im Vakuum (weniger als $10^{-5}$ mbar). Nach der Anregung (definiert als t = 0) wird der zeitliche Verlauf der Intensität der emittierten Photolumineszenz gemessen. Die Photolumineszenz zeigt am Anfang einen steilen Abfall, der auf die prompte Fluoreszenz der TADF-Verbindung zurückzuführen ist. Im weiteren zeitlichen Verlauf ist ein langsamerer Abfall zu beobachten, die verzögerte Fluoreszenz (siehe z. B. H. Uoyama et al., Nature, vol. 492, no. 7428, 234-238, 2012 sowie K. Masui et al., Organic Electronics, vol. 14, no. 11, pp. 2721-2726, 2013). Die Abklingzeit $t_a$ im Sinne dieser Anmeldung ist die Abklingzeit der verzögerten Fluoreszenz und wird wie folgt bestimmt: Man wählt einen Zeitpunkt $t_d$ zu dem die prompte Fluoreszenz deutlich unter die Intensität der verzögerten Fluoreszenz abgeklungen ist, so dass die folgende Bestimmung der Abklingzeit nicht von der prompten Fluoreszenz beeinflusst wird. Diese Wahl kann von einem Fachmann durchgeführt werden und gehört zu dessen allgemeinem Fachwissen. Für die Messdaten ab dem Zeitpunkt $t_d$ wird die Abklingzeit ta = te $-t_d$ bestimmt. Dabei ist $t_e$ derjenige Zeitpunkt nach $t = t_d$, bei dem die Intensität erstmals auf 1/e ihres Wertes bei $t = t_d$ abgefallen ist.

## Beispiel 2

### Devicebeispiele

Die Materialien, die für folgenden Beispiele benötigt werden, sind in Tabelle 1 dargestellt. Die zugehörigen HOMO und LUMO Energieniveaus sowie $S_1$ und $T_1$ sind in Tabelle 2 angegeben.

### Vakuumprozessierte OLEDs

Glasplättchen, die mit strukturiertem ITO (Indium Zinn Oxid) der Dicke 50 nm beschichtet sind werden nass gereinigt (Laborspülmaschine, Reiniger Merck Extran), anschließend 15 min. lang bei 250°C in einer Stickstoffatmosphäre ausgeheizt und vor der Beschichtung 130 s lang mit einem Sauerstoffplasma behandelt. Diese mit Plasma behandelten Glasplättchen bilden die Substrate, auf welche die OLEDs aufgebracht werden. Die Substrate verbleiben vor der Beschichtung im Vakuum. Die Beschichtung beginnt spätestens 10 min. nach der Plasmabehandlung.

Die Materialien werden in einer Vakuumkammer thermisch aufgedampft. Dabei besteht die Emissionsschicht immer aus mindestens einem Matrixmaterial (Hostmaterial, Wirtsmaterial) und dem emittierenden Material. Dieses wird dem Matrixmaterial bzw. den Matrixmaterialien durch Coverdampfung in einem bestimmten Volumenanteil beigemischt. Eine Angabe wie IC1(60%):WB1(30%):D1(10%) bedeutet hierbei, dass das Material IC1 in einem Volumenanteil von 60%, WB1 in einem Anteil von 30% und D1 in einem Anteil von 10% in der Schicht vorliegt. Analog kann auch die Elektronentransportschicht aus einer Mischung von zwei Materialien bestehen.

Zur Charakterisierung der OLEDs werden Strom-Spannungs-Leuchtdichtekennlinien gemessen. Die Leuchtdichte wird mit einer kalibrierten Photodiode bestimmt. Weiterhin wird das Elektrolumineszenzspektrum bei einer Leuchtdichte von 1000 cd/$m^2$ gemessen. Hieraus wird unter Annahme einer lambertschen Abstrahlcharakteristik die externe Quanteneffizienz (EQE, gemessen in Prozent) berechnet.

### Beispiel V1 gemäß dem Stand der Technik:

Auf die vorbereiteten Substrate wird durch thermisches Verdampfen die Schichtfolge 40 nm BPA1, danach 60 nm IC1 (60%):WB1(30%):D1(10%), danach 10nm ST1, danach 40 nm ST1(50%):LiQ(50%) und abschließend 100 nm Aluminium als Kathode aufgebracht.

Die Emissionsschicht zeigt eine PLQE von 84% ($\lambda_{exc}$ = 350 nm) und eine Abklingzeit von 4.9 µs ($t_d$ = 6 µs).

Die OLEDs zeigen grüne Emission, 15.1% EQE bei 1000 cd/$m^2$ und benötigen für diese Leuchtdichte 7.2 V Spannung. Von diesem Beispiel werden 64 Bauteile hergestellt. Beim Betrieb mit 20 mA/$cm^2$ über einen Zeitraum von 200 h fallen sieben der Bauteile aus, d.h., sie zeigen keinerlei Emission mehr.

### Beispiel V2 gemäß dem Stand der Technik:

Auf die vorbereiteten Substrate wird durch thermisches Verdampfen die Schichtfolge 40 nm BPA1, danach 60 nm IC1(30%):WB1(60%):D1(10%), danach 10 nm ST1, danach 40 nm ST1(50%):LiQ(50%) und abschließend 100 nm Aluminium als Kathode aufgebracht.

Die Emissionsschicht zeigt eine PLQE von 79% ($\lambda_{exc}$ = 350 nm) und eine Abklingzeit von 5.1 µs ($t_d$ = 7 µs).

Die OLEDs zeigen grüne Emission, 13.9% EQE bei 1000 cd/$m^2$ und benötigen für diese Leuchtdichte 6.8 V Spannung. Von diesem Beispiel werden 64 Bauteile hergestellt. Beim Betrieb mit 20 mA/$cm^2$ über einen Zeitraum von 200 h fallen fünf der Bauteile aus, d.h., sie zeigen keinerlei Emission mehr.

### OLEDs mit aus Lösung prozessierter Emissionsschicht

In den im Folgenden diskutierten Beispielen werden lösungsbasiert und vakuumbasiert aufgebrachte Schichten innerhalb einer OLED kombiniert, so dass die Prozessierung bis einschließlich zur Emissionsschicht aus Lösung und in den darauffolgenden Schichten durch thermisches Verdampfen im Vakuum erfolgt.

Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 20min mit einem UV-Ozon Plasma behandelt und anschließend mit 20 nm PEDOT:PSS beschichtet (Poly(3,4-ethylendioxythiophen)poly(styrolsulfonat), bezogen als CLEVIOS™ P VP AI 4083 von Heraeus Precious Metals GmbH Deutschland, aus wässriger Lösung aufgeschleudert). Diese Substrate werden anschließend bei 180°C für 10 min ausgeheizt.

Auf diese Substrate wird eine Lochtransportschicht der Dicke 20 nm aufgebracht. Sie besteht aus einem Polymer der folgenden Strukturformel,

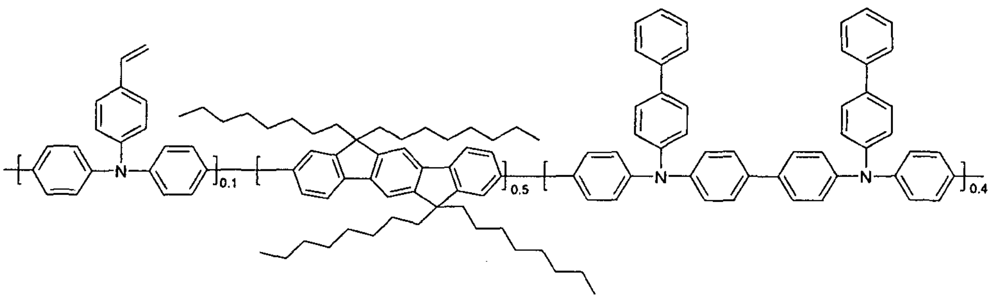

das gemäß WO 2010/097155 synthetisiert wurde. Das Material wird in Toluol gelöst. Der Feststoffgehalt der Lösung liegt bei 5 g/l. Mittels Aufschleudern (Spincoating) wird hieraus in einer Stickstoffatmosphäre eine 20 nm dicke Schicht aufgebracht. Anschließend wird die Probe für 60 Minuten bei 180°C in einer Stickstoffatmosphäre ausgeheizt.

Im Anschluss wird die Emissionsschicht aufgebracht. Diese setzt sich immer aus mindestens einem Matrixmaterial (Hostmaterial, Wirtsmaterial) und einem emittierenden Dotierstoff (Dotand, Emitter) zusammen. Eine Angabe wie IC2(60%):WB1(30%):D1(10%) bedeutet, dass das Material IC2 in einem Gewichtsanteil von 60%, WB1 in einem Gewichtsanteil von 30% und D1 in einem Gewichtsanteil von 10% in der Lösung vorliegt, aus der die Emissionsschicht hergestellt wird. Eine entsprechende Feststoffmischung für die Emissionsschicht wird in Toluol gelöst. Der Feststoffgehalt liegt bei 18 g/l. Die Emissionsschicht wird in einer Stickstoffatmosphäre aufgeschleudert und 10 Minuten bei 180°C in einer Stickstoffatmosphäre ausgeheizt.

Im Anschluss werden die Proben ohne Kontakt zu Luft in eine Vakuumkammer eingebracht und weitere Schichten durch thermisches Verdampfen aufgebracht. Besteht eine solche Schicht aus mehreren Materialien, gilt für die Mischungsverhältnisse der Einzelkomponenten die weiter oben beschriebene Nomenklatur für thermisch aufgedampfte Schichten.

Die OLEDs werden wie für die vakuumprozessierten OLEDs beschrieben charakterisiert.

**Erfindungsgemäßes Beispiel E1:** Für die Emissionsschicht wird eine Feststoffmischung IC2(60%):WB1 (30%):D1 (10%) verwendet. Hieraus wird wie oben beschrieben eine 60 nm dicke Emissionsschicht hergestellt. Anschließend wird eine 10 nm dicke Schicht des Materials ST1 und danach eine 40nm dicke Schicht ST1 (50%):LiQ(50%) durch thermisches Verdampfen im Vakuum aufgebracht. Anschließend wird als Kathode eine 100 nm dicke Aluminiumschicht durch Verdampfen im Vakuum aufgebracht.

Die Emissionsschicht zeigt eine PLQE von 82% ($\lambda_{exc}$ = 350 nm) und eine Abklingzeit von 4.6 $\mu$s ($t_d$ = 5 $\mu$s).

Die OLEDs zeigen grüne Emission, 14.2% EQE bei 1000 cd/m$^2$ und benötigen für diese Leuchtdichte 7.4 V Spannung. Von diesem Beispiel werden 64 Bauteile hergestellt. Beim Betrieb mit 20 mA/cm$^2$ über einen Zeitraum von 200 h fallen zwei der Bauteile aus, d.h., sie zeigen keinerlei Emission mehr.

**Erfindungsgemäßes Beispiel E2:** Die OLED entspricht dem Beispiel E1, mit dem Unterschied, dass statt der Mischung
IC2(60%):WB1(30%):D1(10%) die Mischung
IC2(30%):WB1(60%):D1(10%) verwendet wird.

Die Emissionsschicht zeigt eine PLQE von 75% ($\lambda_{exc}$ = 350 nm) und eine Abklingzeit von 4.7 $\mu$s ($t_d$ = 5 $\mu$s).

Die OLEDs zeigen grüne Emission, 12.9% EQE bei 1000 cd/m$^2$ und benötigen für diese Leuchtdichte 6.9 V Spannung. Von diesem Beispiel werden 64 Bauteile hergestellt. Beim Betrieb mit 20 mA/cm$^2$ über einen Zeitraum von 200 h fällt keines der Bauteile aus.

**Beispiel V3 (gemäß dem Stand der Technik) mit vakuumprozessierter Emissionsschicht**

Die OLED entspricht der des Beispiels E1 mit dem Unterschied, dass die Emissionsschicht vakuumprozessiert ist, d.h., die 60 nm dicke Emissionsschicht IC2(60%):WB1(30%):D1(10%) wird durch Verdampfen im Vakuum hergestellt.

Die Emissionsschicht zeigt eine PLQE von 86% ($\lambda_{exc}$ = 350nm) und eine Abklingzeit von 4.7$\mu$s ($t_d$ = 5$\mu$s).

Die OLEDs zeigen grüne Emission, 13.5% EQE bei 1000 cd/m$^2$ und benötigen für diese Leuchtdichte 7.5 V Spannung. Von diesem Beispiel werden 64 Bauteile hergestellt. Beim Betrieb mit 20 mA/cm$^2$ über einen Zeitraum von 200 h fallen vier der Bauteile aus, d.h., sie zeigen keinerlei Emission mehr.

**Vergleich der Beispiele**

In den Beispielen V1, V2 gemäß dem Stand der Technik und den erfindungsgemäßen Beispielen E1, E2 werden sehr ähnliche Materialien eingesetzt, die für die jeweilige Prozessierungsart angepasst sind. Insbesondere wird in allen Beispielen die Verbindung D1 als TADF Material eingesetzt.

Der Vergleich der Beispiele V1 und E1 bzw. V2 und E2 zeigt, dass sich mit lösungsprozessierten Emissionsschichten vergleichbare Effizienz und Spannung erreichen lassen wie mit vakuumprozessierten. Allerdings ist die Ausfallrate der vakuumprozessierten OLEDs im Betrieb wesentlich höher.

In den Beispielen V3 und E1 werden identische Materialien verwendet mit dem Unterschied, dass in E1 die Emissionsschicht aus Lösung und in V3 durch Verdampfen im Vakuum hergestellt wird. Die Leistungsdaten sind vergleichbar, die OLEDs mit lössungsprozessierte Emissionsschicht zeigen aber deutlich weniger Ausfälle.

**OLEDs mit Emissionsschichten enthaltend Quantum Rods**

Die OLED entspricht dem Beispiel E1, mit dem Unterschied, dass statt der Mischung IC2(60%):WB1(30%):D1(10%) die Mischung IC2(45%):WB1(23%):D1(7%):QRod(25%) verwendet wird. Dabei ist QRod ein rot emittierendes Quantum Rod das einen CdSe Kern mit 3.9 nm Durchmesser enthält, das umgebende 35 nm lange Stäbchen besteht aus CdS. Als Capping Agent wird Octadecylphosphonic Acid verwendet. Die Peakwellenlänge von QRod beträgt 635 nm, die Halbwertsbreite 30 nm. Die Emissionsschicht ohne QRod zeigt eine PLQE von 81% ($\lambda_{exc}$ = 350 nm) und eine Abklingzeit von 4.8 $\mu$s ($t_d$ = 5 $\mu$s).

Die OLED zeigt gelbe Emission, d.h. eine Mischung aus der grünen Emission von D1 und der roten Emission von QRod. Die EQE beträgt 8.4% bei 1000 cd/m$^2$, für diese Leuchtdichte werden 9.4 V Spannung benötigt.

Tabelle 1: Strukturformeln der Materialien für die OLEDs

| | |
|---|---|
| 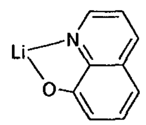 | 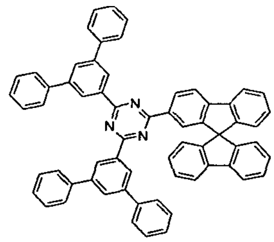 |
| LiQ | ST1 |
| 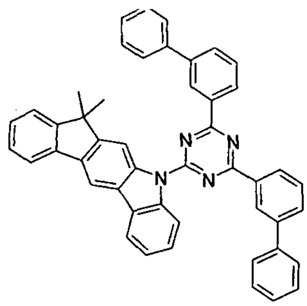 | 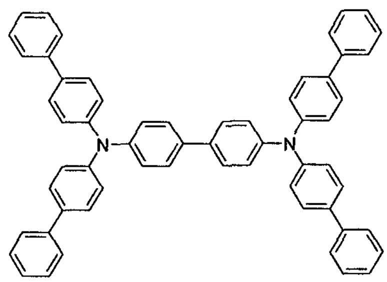 |
| IC1 | BPA1 |
| 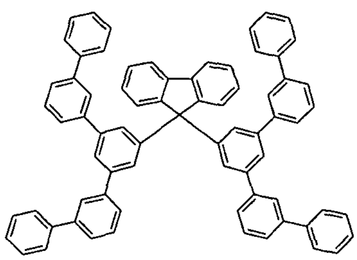 | 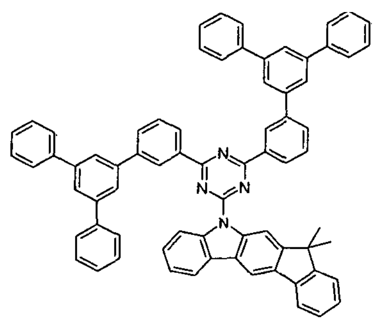 |
| WB1 | IC2 |

(fortgesetzt)

| | |
|---|---|
| 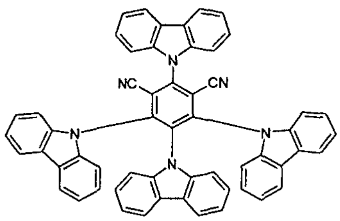 | |
| D1 | |

Tabelle 2: HOMO, LUMO, $T_1$, $S_1$ der relevanten Materialien

| Material | Methode | HOMO (eV) | LUMO (eV) | $S_1$ (eV) | $T_1$ (eV) |
|---|---|---|---|---|---|
| D1 | org. | -6.11 | -3.40 | 2.50 | 2.41 |
| IC1 | org. | -5.80 | -2.83 | 3.12 | 2.70 |
| IC2 | org. | -5.78 | -2.84 | 3.04 | 2.69 |
| WB1 | org. | -6.16 | -2.24 | 3.38 | 2.95 |
| BPA1 | org. | -5.14 | -2.27 | 3.14 | 2.52 |
| ST1 | org. | -6.03 | -2.82 | 3.32 | 2.68 |
| LiQ | M-org. | -5.17 | -2.39 | 2.85 | 2.13 |

## Patentansprüche

1. Formulierung enthaltend wenigstens eine Art organischer lumineszierender Verbindung (TADF-Verbindung) sowie ein organisches Lösungsmittel oder ein Gemisch verschiedener organischer Lösungsmittel, **dadurch gekennzeichnet, dass** die TADF-Verbindung einen Abstand zwischen dem niedrigsten Triplettzustand $T_1$ und dem ersten angeregten Singulettzustand $S_1$ von kleiner oder gleich 0.15 eV aufweist
und wobei
die Formulierung wenigstens eine weitere Komponente enthält, die aus den organischen Halbleitermaterialien, anorganischen Halbleitermaterialien oder aus Quantenpunkten (Quantum Dots) oder Quantenstäbchen (Quantum Rods) ausgewählt wird;
und wobei die Konzentration der TADF-Verbindung in der Formulierung bezogen auf die Gesamtformulierung im Bereich von 1 bis 20 Gew.-% liegt;
und wobei es sich bei der TADF-Verbindung um eine aromatische Verbindung handelt, die sowohl Donor- als auch Akzeptorsubstituenten aufweist, wobei die Donorsubstituenten Diaryl- oder Heteroarylaminogruppen, Carbazole, Indenocarbazole oder Indolocarbazole darstellen und die Aktzeptorgruppen Cyanogruppen, $CF_3$, Triazine, Pyrimidine, Phosphinoxide oder Ketone darstellen.

2. Formulierung gemäß Anspruch 1, **dadurch gekennzeichnet, dass** der Abstand zwischen $S_1$ und $T_1$ der TADF-Verbindung kleiner oder gleich 0.10 eV, bevorzugt kleiner oder gleich 0.08 eV und besonders bevorzugt kleiner oder gleich 0.05 eV ist.

3. Formulierung gemäß Anspruch 1 oder 2, **dadurch gekennzeichnet, dass** die Oberflächenspannung des Lösungsmittels oder der Lösungsmittel wenigstens 28 mN/m, bevorzugt wenigstens 30 mN/m, ganz bevorzugt wenigstens 32 mN/m und ganz besonders bevorzugt wenigstens 35 mN/m beträgt.

4. Formulierung gemäß einem oder mehreren der Ansprüche 1 bis 3, **dadurch gekennzeichnet, dass** die Siede- oder Sublimationstemperatur des Lösungsmittels oder der Lösungsmittel kleiner als 300°C und bevorzugt weniger als 260°C beträgt.

5. Formulierung gemäß einem oder mehreren der Ansprüche 1 bis 4, **dadurch gekennzeichnet, dass** die Viskosität des Lösungsmittel oder die der einzelnen Lösungsmittel eines Lösungsmittelgemischs größer als 3mPa*s und bevorzugt größer als 5mPa*s ist.

6. Formulierung gemäß einem oder mehreren der Ansprüche 1 bis 5, **dadurch gekennzeichnet, dass** das Molekulargewicht des Lösungsmittels oder der im Lösungsmittelgemisch verwendeten Lösungsmittel kleiner oder gleich 1000 g/mol, bevorzugt kleiner oder gleich 700 g/mol, ganz bevorzugt kleiner oder gleich 500 g/mol und besonders bevorzugt kleiner oder gleich 300 g/mol ist.

7. Formulierung gemäß einem oder mehreren der Ansprüche 1 bis 6, **dadurch gekennzeichnet, dass** die Konzentration der TADF-Verbindung in der Formulierung bezogen auf die Gesamtformulierung im Bereich von 3 bis 15 Gew.-% und ganz bevorzugt im Bereich von 5 bis 12 Gew.-% liegt.

8. Formulierung gemäß einem oder mehreren der Ansprüche 1 bis 7, **dadurch gekennzeichnet, dass** das Lösungsmittel ausgewählt wird aus der Gruppe bestehend aus der Gruppe bestehend aus Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethyheneglycolbutylmethyl ether, Triethyleneglycolbutylmethyl ether, Diethyleneglycoldibutyl ether, Triethyleneglycoldimethyl ether, Diethylenglycolmonobutylether, Tripropyleneglycol dimethyl ether, Tetraethyleneglycol dimethyl ether, 2-isopropyl naphthalin, Pentylbenzol, Hexylbenzol, Heptylbenzol, octylbenzol, 1,1-Bis(3,4-Dimethylphenyl)ethan oder aus Mischungen dieser Lösemittel.

9. Formulierung gemäß einem oder mehreren der Ansprüche 1 bis 8, **dadurch gekennzeichnet, dass** die Formulierung wenigstens ein organisches Matrixmaterial enthält, wobei es sich bei den organischen Matrixmaterialien bevorzugt um Carbazole, Indenocarbazole, Indolocarbazole, Triazine, Pyrimidine, Lactame, Ketone, Phosphinoxide, Triphenylene, Diarylfluorene, insbesondere 9,9-Diarylfluorene, oder Dibenzofurane handelt

10. Formulierung gemäß einem oder mehreren der Ansprüche 1 bis 9, **dadurch gekennzeichnet, dass** die Formulierung ein Matrixmaterial enthält wobei für das lowest unoccupied molecular orbital (LUMO) der TADF-Verbindung LUMO(TADF) und das highest occupied molecular orbital (HOMO) der Matrix HOMO(Matrix) gilt:
[LUMO(TADF) - HOMO(Matrix)] ist größer oder gleich [$S_1$(TADF) - 0.4 eV],
wobei $S_1$(TADF) der erste angeregte Singulettzustand $S_1$ der TADF-Verbindung ist.

11. Formulierung gemäß einem oder mehreren der Ansprüche 1 bis 9, **dadurch gekennzeichnet, dass** die Formulierung ein erstes und ein zweites Matrixmaterial enthält.

12. Formulierung gemäß Anspruch 11, **dadurch gekennzeichnet, dass** es sich bei dem ersten Matrixmaterial um ein elektronentransportierendes Matrixmaterial und bei dem zweiten Matrixmaterial um ein lochtransportierendes Matrixmaterial handelt; bevorzugt handelt es sich bei dem elektronentransportierenden Matrixmaterialen um Triazine, Pyrimidine, Lactame, Ketone, Phosphinoxide; bevorzugt handelt es sich bei den lochtransportierenden Matrixmaterialien um Carbazole, Indenocarbazole, Indolocarbazole.

13. Formulierung gemäß Anspruch 11, **dadurch gekennzeichnet, dass** es sich bei sowohl dem ersten als auch bei dem zweiten Matrixmaterial um elektronentransportierende Matrixmaterialien handelt, die bevorzugt Triazine, Pyrimidine, Lactame, Ketone, Phosphinoxide darstellen.

14. Formulierung gemäß Anspruch 11, **dadurch gekennzeichnet, dass** es sich bei dem ersten Matrixmaterial entweder um ein elektronentransportierendes oder um ein lochtransportierendes Matrixmaterial handelt und dass es sich bei dem zweiten Matrixmaterial um eine Verbindung handelt, die ein band gap (= Bandlücke zwischen HOMO und LUMO) von größer oder gleich 3.5 eV handelt.

15. Verwendung einer Formulierung gemäß einem oder mehreren der Ansprüche 1 bis 14 zur Herstellung einer oder mehrere Schichten einer organischen elektronischen Vorrichtung aus Lösung.

16. Verfahren zur Herstellung einer organischen elektronischen Vorrichtung, **dadurch gekennzeichnet, dass** wenigstens eine Schicht der elektronischen Vorrichtung mit Hilfe einer Formulierung gemäß einem oder mehreren der Ansprüche 1 bis 14 aus Lösung hergestellt wird.

**17.** Verfahren gemäß Anspruch 16, **dadurch gekennzeichnet, dass** es sich bei der elektronischen Vorrichtung um eine organische elektrolumineszierende Vorrichtung handelt, bevorzugt handelt es sich um eine OLED.

## Claims

**1.** Formulation comprising at least one type of organic luminescent compound (TADF compound) and an organic solvent or a mixture of various organic solvents, **characterised in that** the TADF compound has a separation between the lowest triplet state $T_1$ and the first excited singlet state $S_1$ of less than or equal to 0.15 eV
and where
the formulation comprises at least one further component selected from organic semiconductor materials, inorganic semiconductor materials or from quantum dots or quantum rods;
and where the concentration of the TADF compound in the formulation, based on the entire formulation, is in the range from 1 to 20% by weight,
and where the TADF compound is an aromatic compound which contains both donor and acceptor substituents, where the donor substituents are diaryl- or heteroarylamino groups, carbazoles, indenocarbazoles or indolocarbazoles and the acceptor groups are cyano groups, $CF_3$, triazines, pyrimidines, phosphine oxides or ketones.

**2.** Formulation according to Claim 1, **characterised in that** the separation between $S_1$ and $T_1$ of the TADF compound is less than or equal to 0.10 eV, preferably less than or equal to 0.08 eV and particularly preferably less than or equal to 0.05 eV.

**3.** Formulation according to Claim 1 or 2, **characterised in that** the surface tension of the solvent or solvents is at least 28 mN/m, preferably at least 30 mN/m, very preferably at least 32 mN/m and very particularly preferably at least 35 mN/m.

**4.** Formulation according to one or more of Claims 1 to 3, **characterised in that** the boiling or sublimation point of the solvent or solvents is less than 300°C and preferably less than 260°C.

**5.** Formulation according to one or more of Claims 1 to 4, **characterised in that** the viscosity of the solvent or that of the individual solvents of a solvent mixture is greater than 3 mPa*s and preferably greater than 5 mPa*s.

**6.** Formulation according to one or more of Claims 1 to 5, **characterised in that** the molecular weight of the solvent or of the solvents used in the solvent mixture is less than or equal to 1000 g/mol, preferably less than or equal to 700 g/mol, very preferably less than or equal to 500 g/mol and particularly preferably less than or equal to 300 g/mol.

**7.** Formulation according to one or more of Claims 1 to 6, **characterised in that** the concentration of the TADF compound in the formulation, based on the entire formulation, is in the range from 3 to 15% by weight and very preferably in the range from 5 to 12% by weight.

**8.** Formulation according to one or more of Claims 1 to 7, **characterised in that** the solvent is selected from the group consisting of toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, □-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)-ethane or mixtures of these solvents.

**9.** Formulation according to one or more of Claims 1 to 8, **characterised in that** the formulation comprises at least one organic matrix material, where the organic matrix materials are preferably carbazoles, indenocarbazoles, indolocarbazoles, triazines, pyrimidines, lactams, ketones, phosphine oxides, triphenylenes, diarylfluorenes, in particular 9,9-diarylfluorenes, or dibenzofurans.

**10.** Formulation according to one or more of Claims 1 to 9, **characterised in that** the formulation comprises a matrix material, where the following applies for the lowest unoccupied molecular orbital (LUMO) of the TADF compound LUMO(TADF) and the highest occupied molecular orbital (HOMO) of the matrix HOMO(matrix):
[LUMO(TADF) - HOMO(matrix)] is greater than or equal to [$S_1$(TADF) - 0.4 eV],
where $S_1$(TADF) is the first excited singlet state $S_1$ of the TADF compound.

**11.** Formulation according to one or more of Claims 1 to 9, **characterised in that** the formulation comprises a first matrix material and a second matrix material.

**12.** Formulation according to Claim 11, **characterised in that** the first matrix material is an electron-transporting matrix material and the second matrix material is a hole-transporting matrix material; the electron-transporting matrix materials are preferably triazines, pyrimidines, lactams, ketones or phosphine oxides; the hole-transporting matrix materials are preferably carbazoles, indenocarbazoles or indolocarbazoles.

**13.** Formulation according to Claim 11, **characterised in that** both the first matrix material and the second matrix material are electron-transporting matrix materials, which are preferably triazines, pyrimidines, lactams, ketones or phosphine oxides.

**14.** Formulation according to Claim 11, **characterised in that** the first matrix material is either an electron-transporting matrix material or a hole-transporting matrix material and **in that** the second matrix material is a compound which has a band gap between HOMO and LUMO of greater than or equal to 3.5 eV.

**15.** Use of a formulation according to one or more of Claims 1 to 14 for the production of one or more layers of an organic electronic device from solution.

**16.** Process for the production of an organic electronic device, **characterised in that** at least one layer of the electronic device is produced from solution with the aid of a formulation according to one or more of Claims 1 to 14.

**17.** Process according to Claim 16, **characterised in that** the electronic device is an organic electroluminescent device, preferably an OLED.

## Revendications

**1.** Formulation comprenant au moins un type de composé luminescent organique (composé TADF) et un solvant organique ou un mélange de divers solvants organiques, **caractérisée en ce que** le composé TADF présente une séparation entre l'état triplet le plus bas $T_1$ et le premier état singulet excité $S_1$ qui est inférieure ou égale à 0,15 eV et dans laquelle :

la formulation comprend au moins un autre composant qui est sélectionné parmi les matériaux semiconducteurs organiques, les matériaux semiconducteurs inorganiques ou parmi les points quantiques ou les barres quantiques ; et dans laquelle :
la concentration du composé TADF dans la formulation, sur la base de la formulation complète, s'inscrit dans la plage qui va de 1 % à 20 % en poids ; et dans laquelle :
le composé TADF est un composé aromatique qui contient à la fois des substituants donneurs et accepteurs, dans laquelle les substituants donneurs sont les groupes diaryl- ou hétéroarylamino, les carbazoles, les indénocarbazoles ou les indolocarbazoles et les groupes accepteurs sont les groupes cyano, le $CF_3$, les triazines, les pyrimidines, les oxydes de phosphine ou les cétones.

**2.** Formulation selon la revendication 1, **caractérisée en ce que** la séparation entre $S_1$ et $T_1$ du composé TADF est inférieure ou égale à 0,10 eV, de préférence inférieure ou égale à 0,08 eV et de façon particulièrement préférable, inférieure ou égale à 0,05 eV.

**3.** Formulation selon la revendication 1 ou 2, **caractérisée en ce que** la tension de surface du solvant ou des solvants est d'au moins 28 mN/m, de préférence d'au moins 30 mN/m, de façon très préférable d'au moins 32 mN/m et de façon très particulièrement préférable, d'au moins 35 mN/m.

4. Formulation selon une ou plusieurs des revendications 1 à 3, **caractérisée en ce que** le point d'ébullition ou de sublimation du solvant ou des solvants est inférieur à 300°C et de préférence, est inférieur à 260°C.

5. Formulation selon une ou plusieurs des revendications 1 à 4, **caractérisée en ce que** la viscosité du solvant ou celle des solvants individuels d'un mélange de solvants est supérieure à 3 mPa*s et de préférence, est supérieure à 5 mPa*s.

6. Formulation selon une ou plusieurs des revendications 1 à 5, **caractérisée en ce que** le poids moléculaire du solvant ou des solvants qui sont utilisés dans le mélange de solvants est inférieur ou égal à 1000 g/mol, de préférence, il est inférieur ou égal à 700 g/mol, de façon très préférable, il est inférieur ou égal à 500 g/mol et de façon particulièrement préférable, il est inférieur ou égal à 300 g/mol.

7. Formulation selon une ou plusieurs des revendications 1 à 6, **caractérisée en ce que** la concentration du composé TADF dans la formulation, sur la base de la formulation complète, s'inscrit dans la plage qui va de 3 % à 15 % en poids et de façon très préférable, dans la plage qui va de 5 % à 12 % en poids.

8. Formulation selon une ou plusieurs des revendications 1 à 7, **caractérisée en ce que** le solvant est sélectionné parmi le groupe qui est constitué par le toluène, l'anisole, le o-, m- ou p-xylène, le benzoate de méthyle, le mésitylène, la tétraline, le vératrol, le THF, le méthyl-THF, THP, le chlorobenzène, le dioxane, le phénoxytoluène, en particulier le 3-phénoxytoluène, le (-)-fenchone, le 1,2,3,5-tétraméthylbenzène, le 1,2,4,5-tétraméthylbenzène, le 1-méthylnaphtalène, le 2-méthylbenzothiazole, le 2-phénoxyéthanol, le 2-pyrrolidinone, le 3-méthylanisole, le 4-méthylanisole, le 3,4-diméthylanisole, le 3,5-diméthylanisole, l'acétophénone, le □-terpinéol, le benzothiazole, le benzoate de butyle, le cumène, le cyclohexanol, le cyclohexanone, le cyclohexylbenzène, la décaline, le dodécylbenzène, le benzoate d'éthyle, l'indane, le benzoate de méthyle, le NMP, le p-cymène, le phénétol, le 1,4-diisopropylbenzène, l'éther dibenzylique, le butylméthyléther de diéthylène glycol, le butylméthyléther de triéthylène glycol, le dibutyléther de diéthylène glycol, le diméthyléther de triéthylène glycol, le monobutyléther de diéthylène glycol, le diméthyléther de tripropylène glycol, le diméthyléther de tétraéthylène glycol, le 2-isopropylnaphtalène, le pentylbenzène, l'hexylbenzène, l'heptylbenzène, l'octylbenzène, le 1,1-bis(3,4-diméthylphényl)éthane ou des mélanges de ces solvants.

9. Formulation selon une ou plusieurs des revendications 1 à 8, **caractérisée en ce que** la formulation comprend au moins un matériau de matrice organique, dans lequel les matériaux de matrice organiques sont de préférence les carbazoles, les indénocarbazoles, les indolocarbazoles, les triazines, les pyrimidines, les lactams, les cétones, les oxydes de phosphine, les triphénylènes, les diarylfluorènes, en particulier les 9,9-diarylfluorènes, ou les dibenzofuranes.

10. Formulation selon une ou plusieurs des revendications 1 à 9, **caractérisée en ce que** la formulation comprend un matériau de matrice, dans laquelle ce qui suit s'applique pour l'orbitale moléculaire non occupée la plus basse (LUMO) du composé TADF LUMO(TADF) et pour l'orbitale moléculaire occupée la plus haute (HOMO) de la matrice HOMO(matrice) :
[LUMO(TADF) - HOMO(matrice)] est supérieur ou égal à [$S_1$(TADF) - 0,4 eV],
où $S_1$(TADF) est le premier état singulet excité $S_1$ du composé TADF.

11. Formulation selon une ou plusieurs des revendications 1 à 9, **caractérisée en ce que** la formulation comprend un premier matériau de matrice et un second matériau de matrice.

12. Formulation selon la revendication 11, **caractérisée en ce que** le premier matériau de matrice est un matériau de matrice de transport d'électrons et le second matériau de matrice est un matériau de matrice de transport de trous ; les matériaux de matrice de transport d'électrons sont de préférence les triazines, les pyrimidines, les lactams, les cétones ou les oxydes de phosphine ; les matériaux de matrice de transport de trous sont de préférence les carbazoles, les indénocarbazoles ou les indolocarbazoles.

13. Formulation selon la revendication 11, **caractérisée en ce que** le premier matériau de matrice et le second matériau de matrice sont tous deux des matériaux de matrice de transport d'électrons, lesquels sont de préférence les triazines, les pyrimidines, les lactams, les cétones ou les oxydes de phosphine.

14. Formulation selon la revendication 11, **caractérisée en ce que** le premier matériau de matrice est soit un matériau de matrice de transport d'électrons, soit un matériau de matrice de transport de trous et **en ce que** le second matériau de matrice est un composé qui présente une bande interdite entre HOMO et LUMO qui est supérieure ou

égale à 3,5 eV.

**15.** Utilisation d'une formulation selon une ou plusieurs des revendications 1 à 14 pour la fabrication d'une ou de plusieurs couche(s) d'un dispositif électronique organique à partir d'une solution.

**16.** Procédé pour la fabrication d'un dispositif électronique organique, **caractérisé en ce qu'**au moins une couche du dispositif électronique est fabriquée à partir d'une solution à l'aide d'une formulation selon une ou plusieurs des revendications 1 à 14.

**17.** Procédé selon la revendication 16, **caractérisé en ce que** le dispositif électronique est un dispositif électroluminescent organique, de préférence une OLED.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2013154064 A
- WO 2013161437 A
- WO 2013081088 A
- WO 2013011954 A
- WO 2011076325 A1
- EP 13001800 A
- EP 13001797 A
- WO 2009124627 A
- EP 1888708 A
- US 6392250 B1
- US 6803720 B2
- US 7294849 B
- US 10796832 B
- US 10656910 B
- US 60578236 B
- US 20070034833 A1
- US 20050109989 A1
- US 20130135558 A1
- WO 2010097155 A


### In der Beschreibung aufgeführte Nicht-Patentliteratur


- **M. A. BALDO et al.** *Appl. Phys. Lett.,* 1999, vol. 75, 4-6
- **H. UOYAMA et al.** *Nature,* 2012, vol. 492, 234
- **H.UOYAMA et al.** *Nature,* 2012, 234-238
- **J.ZIEGLER et al.** *Adv.Mater.,* 2008, 44068-4073
- **TANAKA et al.** *Chemistry of Materials,* 2013, vol. 25 (18), 3766
- **ZHANG et al.** *Nature Photonics advance online publication,* 2014, vol. 1
- **SEREVICIUS et al.** *Physical Chemistry Chemical Physics,* 2013, vol. 15 (38), 15850
- **YOUN LEE et al.** *Applied Physics Letters,* 2012, vol. 101 (9), 093306
- **NASU et al.** *ChemComm,* 2013, vol. 49, 10385
- **C. M. HANSEN.** Hansen Solubility Parameters: A User's Handbook. Taylor and Francis Group, LLC, 2007
- *J. Am. Chem. Soc.,* 1993, vol. 115 (19), 8706-8715
- *Science,* 1996, vol. 271, 933
- *J. Am. Chem. Soc.,* 1997, vol. 30, 7019-7029
- *J. Am. Chem. Soc.,* 1993, vol. 115, 8706
- **X. PENG et al.** *J. Am. Chem. Soc.,* 1997, vol. 119, 7019-7029
- *J. Am. Chem. Soc.,* 1997, vol. 119, 7019-7029
- **H. UOYAMA et al.** *Nature,* 2012, vol. 492 (7428), 234-238
- **K. MASUI et al.** *Organic Electronics,* 2013, vol. 14 (11), 2721-2726